# EUROPEAN PATENT APPLICATION

(11) **EP 2 727 596 A1**
(43) Date of publication of application: **07.05.2014**
(21) Application number: 12190570.7
(22) Date of filing: 30.10.2012
(51) Int. Cl.: A61K 31/713, G01N 33/68

(54) **eEF2/eEF2K as therapeutic target for treating TNF-alpha-related diseases**

(71) Applicant: Fundación Centro Nacional de Investigaciones Cardiovasculares Carlos III (CNIC), 28029 Madrid (ES)
(72) Inventor: Sabio Buzo, Guadalupe, 28029 Madrid (ES); González Terán, Bárbara, 28029 Madrid (ES)
(74) Representative: Illescas, Manuel

(57) **Abstract**

**eEF2/eEF2k as therapeutic target for treating TNF-alpha-related diseases.** The present invention comprises inhibitors of eEF2 and/or activators of eEF2 kinase for use in the treatment of TNF-alpha-related diseases, preferably liver diseases. Consequently this invention can be included either in the pharmaceutical, biotechnological or medical field.

## Description

### FIELD OF THE INVENTION

The present invention comprises inhibitors of eEF2 and/or activators of eEF2 kinase for use in the treatment of TNF-alpha-related diseases, preferably liver diseases. Consequently this invention can be included either in the pharmaceutical, biotechnological or medical field.

### STATE OF THE ART

Infection and tissue injury trigger a protective inflammatory response that mobilizes a variety of effector mechanisms to contain and eliminate the injurious agent (1). Hepatic and circulating inflammatory cytokines initiate a positive feedback loop on the innate immune system (2); although cytokine production protects against pathogenic stimuli, it can cause more damage than the initiating event if its magnitude and duration are not strictly controlled by intrinsic negative regulators (1).

The cytokine TNFα has been identified as a key regulator of the inflammatory response in animal models of fulminant hepatitis (3). Elevated concentrations of this pro-inflammatory cytokine are also found in acute and chronic inflammatory conditions, including trauma, sepsis, and rheumatoid arthritis (4). Upon binding to its receptors, TNFα initiates several intracellular signaling cascades that influence cell survival, death, differentiation, proliferation and migration (5, 6).

TNFα signaling is particularly important in the liver. While it mediates hepatocyte survival and proliferation, it is also implicated in liver failure since it triggers hepatocyte apoptosis and leads to up-regulation of key adhesion molecules and chemokines involved in leukocyte migration and infiltration (6, 7). A well-established mouse model of acute liver failure and septic shock is the *in vivo* administration of bacterial LPS and D-galactosamine (D-Gal), which strongly induce endogenous TNFα and other cytokine production that causes liver damage (8, 9).

TNFα signaling can be mediated by protein kinases and phosphatases, in particular the mitogen-activated protein kinase (MAPK) family members: ERK, JNK and p38 MAPK. The p38 MAPK pathway transduces a variety of extracellular signals regulating cellular responses to stress, being implicated in cell proliferation, differentiation and apoptosis (10, 11). Four p38 MAPK family members have been identified: p38α, β, γ and δ, also known as stress-activated kinase 2 SAPK2a, SAPK2b, SAPK3 and SAPK4 (10, 12, 13). The p38MAPK family can be further divided into two subsets, with p38α and p38β in one group and p38γ and p38δ in the other. All these kinases are activated by double phosphorylation mediated by the upstream kinases MKK3 and MKK6 (14, 15). To date, the best-characterized p38 isoform is p38α. This isoform is required for macrophage inflammatory responses (1), but in hepatocytes prevents endotoxin-induced liver damage (16, 17). Studies using inhibitors suggested that p38α and p38β cooperate in inflammatory processes; however, p38β knockout mice show no differences in several *in vivo* and *in vitro* models of inflammation (18). Less is known about the role of the p38γ and p38δ MAPK. Hence p38δ is known to phosphorylate eEF2 kinase (eEF2K), inhibiting its activity (19). Enzyme eEF2K blocks mRNA translation into protein through an inhibitory phosphorylation of eukaryotic elongation factor 2 (eEF2).

The rate-limiting step in mRNA translation is thought to be the initiation step; however, far less is known about the regulation of the elongation step of protein biosynthesis (20). The process of peptide-chain elongation is, at least partially, regulated by eEF2 phosphorylation/dephosphorylation events induced by diverse stimuli (21). Moreover, the phosphorylation state of the upstream kinase eEF2K can be modulated by signaling molecules that regulate its activity, such as mTOR, MAPKs, and also including p38 kinases (19, 21-23).

As explained above, TNFα is the key regulator of a high amount of pathologic processes. The present invention provides a therapeutic target where drugs can be directed in order to inhibit the expression or elongation of TNFα, thus becoming candidates for treating TNFα-related-diseases, preferably liver diseases, without showing hepatotoxicity.

### DESRIPTION OF THE INVENTION

### Brief description of the invention

The present invention shows that p38δ and p38γ expressed in myeloid cells, promote eEF2 activity and in consequence TNFα production and the subsequent inflammatory response. By blocking p38γ/δ activity in myeloid macrophages, it was possible to suppress TNFα-relates-diseases, such as hepatitis caused by LPS-induced TNFα expression. The expression of p38γ/δ MAPK proteins is required for the elongation of nascent TNFα in macrophages. MKK3/6-p38γ/δ pathway mediates an inhibitory phosphorylation of eEF2k that promotes eEF2 activation (dephosphorylation) and subsequent specific TNFα elongation. Consequently, the present invention identifies a new signaling pathway that regulates TNFα production, preferably in LPS-induced liver damage, and provides cell-specific therapeutic targets for treating TNFα-related-diseases, preferably liver diseases, in which TNFα production is involved.

Moreover, the present invention illustrates how the production of TNFα is avoided by blocking p38γ/δ activity in myeloid macrophages, being able to suppress TNFα-related-diseases, such as hepatitis caused by LPS-induced TNFα expression. In a preferred embodiment of the invention the production, expression or elongation of TNFα is avoided by means of the inhibition of eEF2 and/or activation of eEF2k. Furthermore, it is important to consider that eEF2k is not the only substrate regulated by p38γ/δ, because p38γ/δ encompass other possible substrates for example: Tau, PKD1, Stathmin, P53, alpha1-syntrophin, SAP90, SAP97, MyoD, Sap or ATF2. So, it can be said that this is a selection invention, where eEF2k has been particularly selected from all the possible substrates of p38γ/δ.

In other words, the preferred embodiment of the invention refers to the use of eEF2 or eEF2k as therapeutic targets for treating TNFα-related-diseases, preferably liver diseases caused by LPS endotoxin. LPS is implicated in the pathogenesis of acute liver disease through induction of synthesis of tumor necrosis factor TNFα, a key determinant of the outcome of acute liver failure during septic shock.

Consequently, the first embodiment of the present invention refers to inhibitors of eEF2 and/or activators of eEF2k for use in the treatment of TNF-alpha-related diseases. Alternatively, this first embodiment may be defined as the use of inhibitors of eEF2 and/or activators of eEF2k for the manufacture of a pharmaceutical composition for treating TNF-alpha-related diseases.

For the purpose of the present invention the following terms are defined:
- Inhibitor of eEF2: any agent or substance that slows, interferes, reduces or suppresses the activity or expression of eEF2. Any inhibitor of eEF2, for example shRNA (shEF2) (C1 RMM4431-98752395, C2 RMM 4431-101293216 or C3 RMM 4431-101293680) Thermo Scientific, Open-biosystems; sordarin (CAS 11076-17-89) or ciclohexamide (CAS 66-81-9), can be used in the present invention, but shRNA (C3 RMM 4431-101293680) is used as proof of concept to illustrate the invention.
- Activator of eEF2k: any agent or substance that stimulates or increases the activity of eEF2k. The term activator of eEF2k also includes any inhibitor of p38γ/δ MAPK. Any activator of eEF2k or inhibitor of p38γ/δ MAPK can be used, but BIRB796 (CAS 285983-48-4) is used in the present invention as proof of concept to exemplify the invention.
- TNF-alpha-related diseases: any disease disorder or pathological process in which this cytokine is involved, preferably in systemic inflammation. It is believed that the group of diseases, disorders or pathological processes caused by TNF-alpha pertains to the common general knowledge, and any of these diseases, disorders or pathological processes can be treated by means of the administration of the inhibitors of eEF2 and/or activators of eEF2k defined in the present invention. Some examples of TNF-alpha-related diseases are rheumatoid arthritis, ankylosing spondylitis, psoriasis, ischaemic brain injury, traumatic injury, cardiovascular diseases such as atherosclerosis, myocardial infarction, heart failure, myocarditis and cardiac allograft rejection, respiratory diseases such as chronic bronchitis, obstructive pulmonary disease, acute respiratory distress syndrome and asthma, renal disease such as ischaemic renal injury and renal transplant rejection and glomerulonephritis, and diabetes. Liver diseases (hepatitis, steatosis, liver failure), particularly LPS-induced liver diseases, are treated in the preset invention as proof of concept to characterize a disease regulated by TNF-alpha.

Consequently, in a preferred embodiment, the invention refers to inhibitors of eEF2 and/or activators of eEF2k for use in the treatment of liver diseases, rheumatoid arthritis, ankylosing spondylitis, psoriasis, ischaemic brain injury, traumatic injury, cardiovascular diseases, respiratory diseases, renal diseases or diabetes. In a particularly preferred embodiment the present invention refers to inhibitors of eEF2 and/or activators of eEF2k for use in the treatment of LPS-induced liver diseases, cardiovascular diseases selected from: atherosclerosis, myocardial infarction, heart failure, myocarditis and cardiac allograft rejection; respiratory diseases selected from: chronic bronchitis, obstructive pulmonary disease, acute respiratory distress syndrome and asthma; or renal diseases selected from: ischaemic renal injury, renal transplant rejection and glomerulonephritis. The present invention also comprises the use of inhibitors of eEF2 and/or activators of eEF2k for the manufacture of pharmaceutical composition for treating TNFα-related-diseases.

On the other hand the present invention refers to pharmaceutical compositions comprising inhibitors of eEF2 and/or activators of eEF2k, and pharmaceutically acceptable carriers, for treating TNFα-related-diseases. The term pharmaceutically acceptable carrier refers to any substance, known in the art, which serve as mechanism to improve the delivery and the effectiveness of the inhibitors of eEF2 and/or activators of eEF2k. Said carriers can be used in sundry drug delivery systems such as: controlled-release technology to prolong in vivo drug actions; decrease drug metabolism, and reduce drug toxicity. Carriers can be also used for increasing the effectiveness of drug delivery to the target sites of pharmacological actions. The compounds used in the present invention may be administered in the form of liposomes, microspheres made of the biodegradable polymer poly(lactic-co-glycolic) acid, albumin microspheres, synthetic polymers (soluble), nanofibers, protein-DNA complexes, protein conjugates, erythrocytes, virosomes or beta1,3-D-glucan-encapsulated siRNA particles (GeRPs).

The second embodiment of the present invention refers to a method for treating TNFα-related-diseases that comprises the administration of an effective amount of inhibitors of eEF2 and/or activators of eEF2k to a patient in need. The term effective amount refers to the minimum amount able to treat or reverse said diseases.

The third embodiment of the present invention refers to an i*n vitro* or *in vivo* method for identifying chemical compounds able to inhibit eEF2 and/or activate eEF2k that comprises contacting said compounds with cells expressing eEF2, stimulating the cell with LPS and determining whether TNFα is infra-expressed as compared with the control. The threshold was 2000 pg/ml for macrophages (1.10⁶/ml) stimulated with 10µg/ml of LPS. The threshold was 3000 pg/ml for the *in vivo* assay and mice injected with DGal+LPS. When the value of TNFα is below said threshold, it is considered that TNFα is infra-expressed (see **Example 11).**

### Detailed description of the invention

The present invention shows that protein kinases p38γ/δ mediate the development of LPS-induced acute hepatitis by acting within a protein kinase signaling network that regulates the production of TNFα by hematopoietic cells. MKK3 and MKK6 activate p38γ/δ, which in turn phosphorylate and inactivate eEF2k. Once eEF2K is inactivated, eEF2 is dephosphorylated and activated; allowing the translational elongation of nascent TNFα. The present invention shows for the first time that eEF2k/eEF2 control cytokine production in myeloid cells, showing how acute inflammatory responses can be controlled through tight regulation of translational elongation.

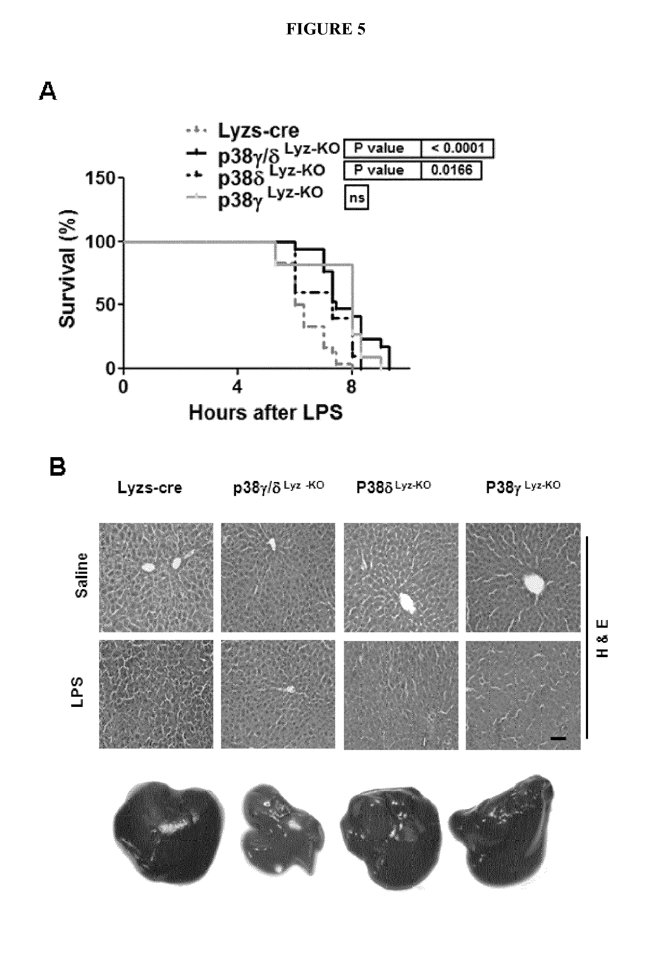

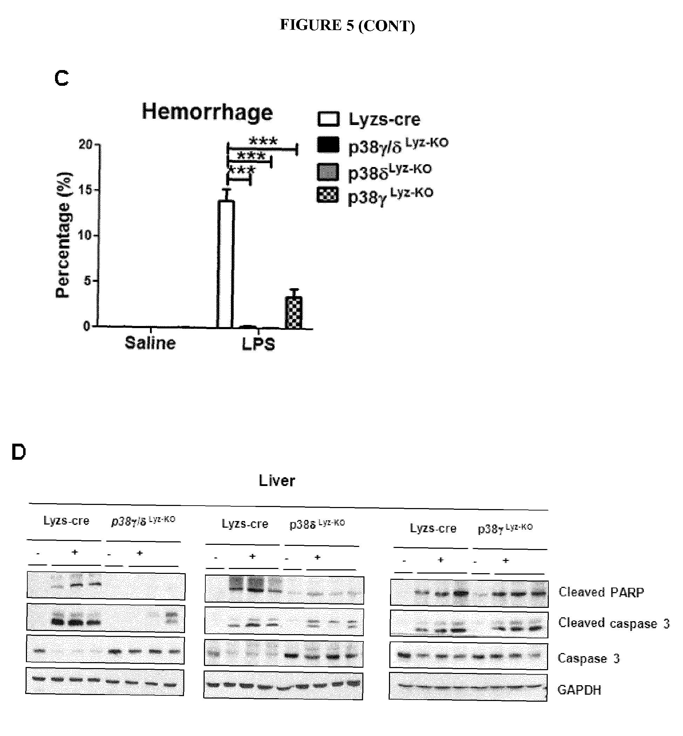

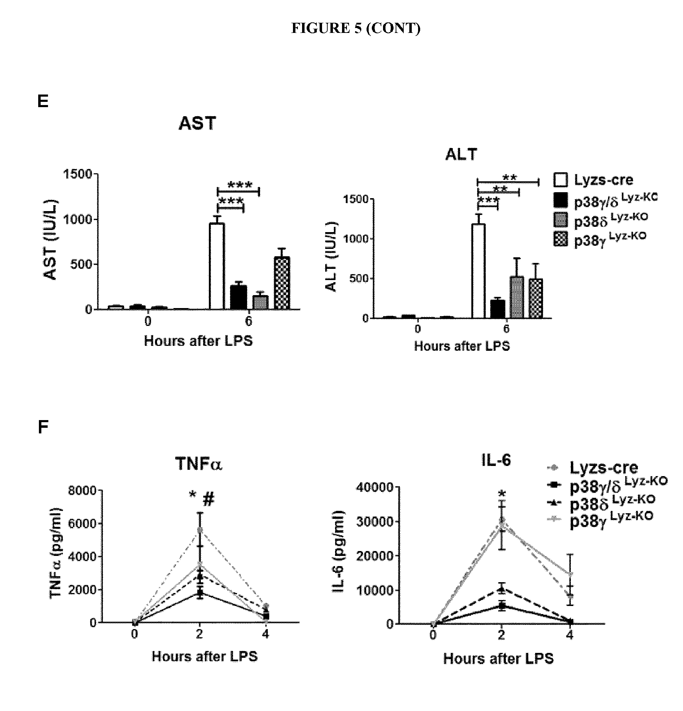

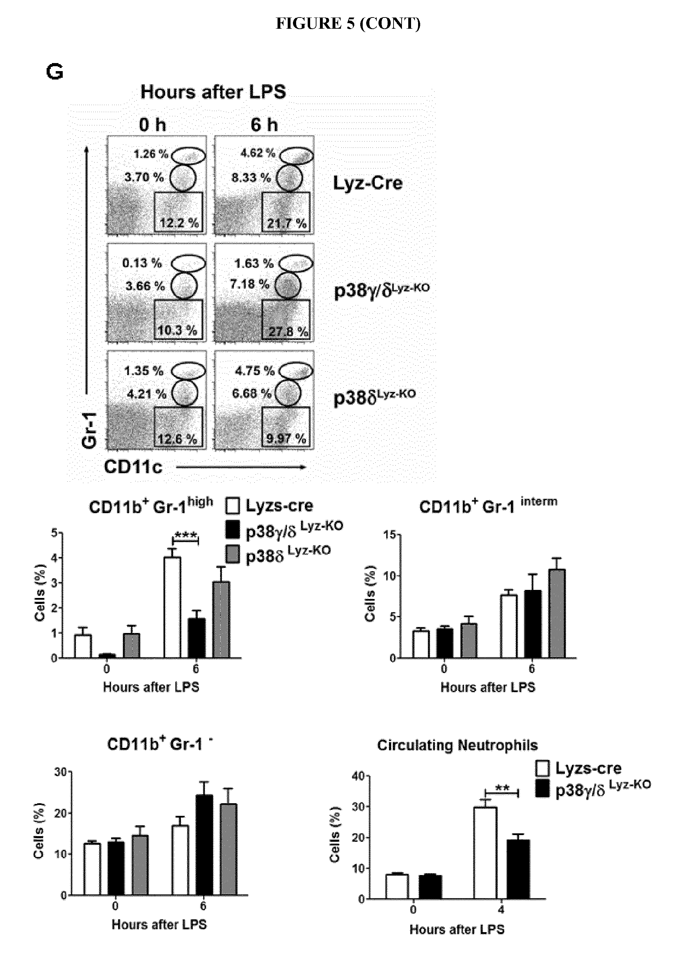

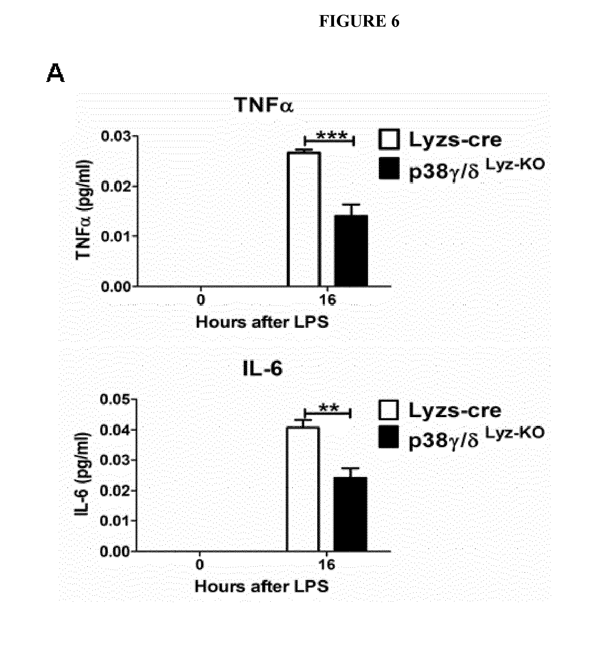

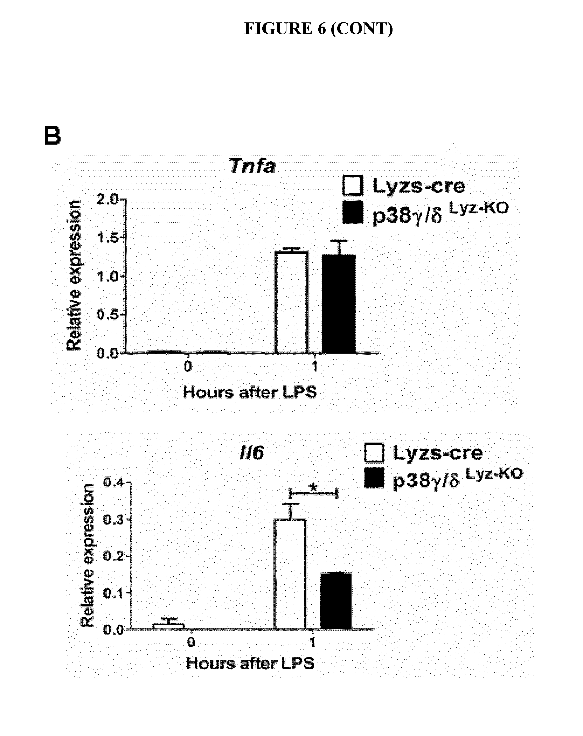

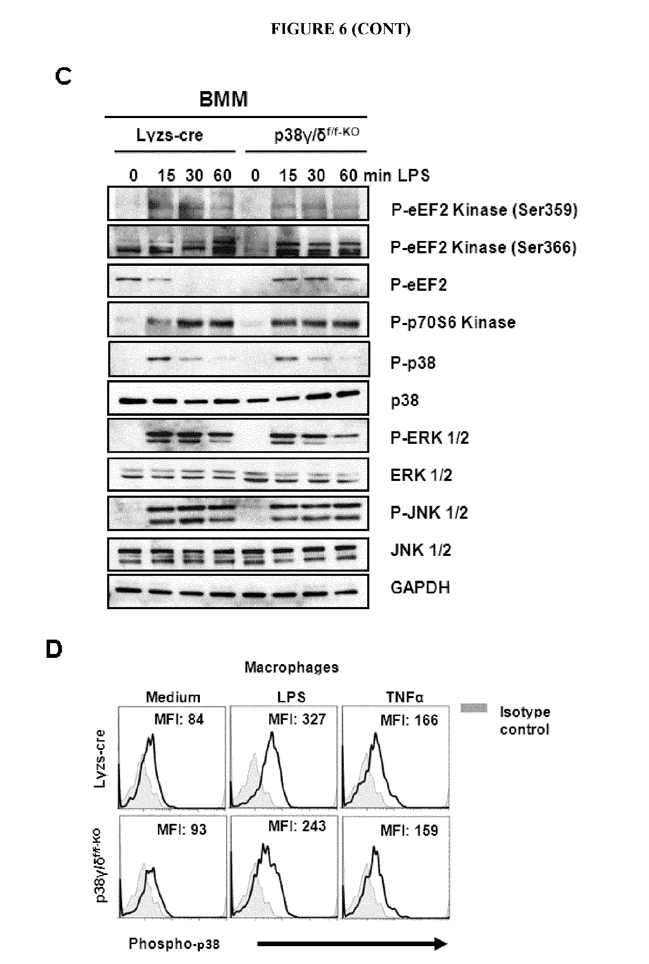

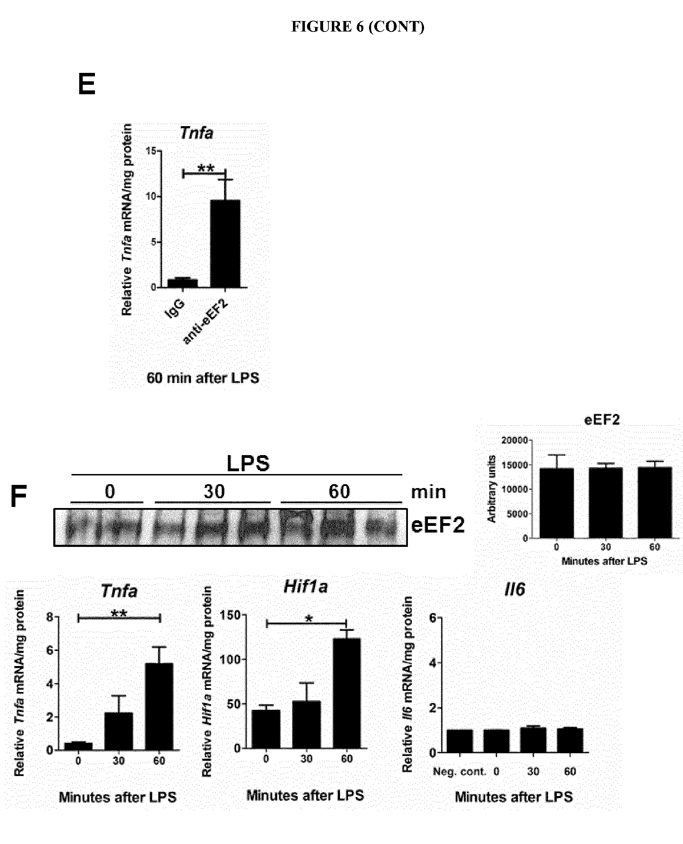

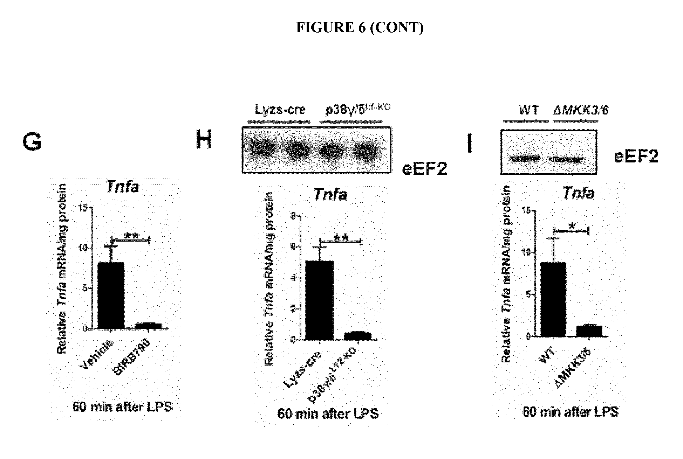

Myeloid cells such as Kupffer cells and other macrophages are well known to be a critical source of TNFα in LPS-induced hepatitis. This specific role of hematopoietic cells is consistent with the results shown below, demonstrating that the protective effects of MKK3/6-deficiency are also present in irradiated WT mice reconstituted with ΔMKK3/6 bone marrow (**Figure 5**). Furthermore, this protection was also observed in mice specifically lacking p38γ/δ in the myeloid compartment (**Figure 6****).** Two cell types of special relevance in acute hepatitis are neutrophils and monocytes/macrophages (including Kupffer cells), and both these bone marrow-derived cell populations are essential for the innate immune response. Monocytes/macrophages bind to microbial constituents (such as LPS and cell wall constituents of Gram-positive bacteria), producing large amounts of pro- and anti-inflammatory cytokines. Neutrophil recruitment and activation occurs through TNFα-mediated chemokine production. This is evidenced by the present invention demonstrating that low expression of TNFα in ΔMKK3/6 or p38γ/δ^{Lyz-KO} mice results in decreased chemokine production and neutrophil migration, an effect reversed by injection with TNFα.

Previous reports have identified p38α as a key kinase involved in TNFα production. p38α MAPK-deficiency in macrophages results in decreased TNFα production with a modest effect on IL6 expression. However, specific inhibition of this kinase has been shown to be hepatotoxic, hindering its clinical use. For example, the p38α inhibitor AMG 548 showed >85% inhibition of ex-vivo LPS-induced TNFα in healthy males, but its production and clinical use was suspended due to random liver enzyme elevations that were not dose or exposure dependent. The present invention shows that specific inhibition of p38α/β with SB203580 intensifies D-Gal+LPS-induced liver damage, whereas BIRB796 that inhibits all four p38 isoforms (α/β/γ/δ) improves liver condition, including a reduction in apoptosis and necrosis. The toxicity associated with SB203580 is most probably caused by inhibition of p38α in hepatocytes, since mice with specific p38α deficiency in hepatocytes have increased JNK activity and increased susceptibility to liver damage. In clinical use, BIRB796 also produces some liver enzyme elevations (24), most likely because of inhibition of p38α. The present invention indicates that the inhibitors that specifically target p38γ/δ kinases avoid the adverse effects (hepatotoxicity) found with p38α inhibitors.

In a preferred embodiment, the present invention further shows that p38γ/δ control macrophage production of TNFα by promoting protein elongation during translation by eEF2. Protein synthesis is tightly regulated at transcriptional and post-transcriptional levels. Owing to the relatively long lifetime of mRNA transcripts, transcriptional regulation is commonly involved in slow, long-term cell responses, whereas immediate cell responses require post-transcriptional regulation of mRNA stability or translation. Two physiological situations requiring rapid regulation of protein synthesis are starvation and inflammatory conditions. In starvation conditions, mTOR stops protein synthesis at the elongation step-the step that consumes most metabolic energy- by inhibiting eEF2, thereby preventing cell energy depletion. Less is known about the mechanisms involved in the termination of the inflammatory response once it has achieved its goal. This process needs to be rapidly regulated in order to avoid more tissue damage. The present invention demonstrates that control of protein elongation might be an important mechanism by which cells rapidly shut down production of pro-inflammatory proteins that could extend tissue damage in the body, such as TNFα during an inflammatory response. This might be an important mechanism by which cells tightly regulate cytokine production induced by stress kinases activated during inflammation.

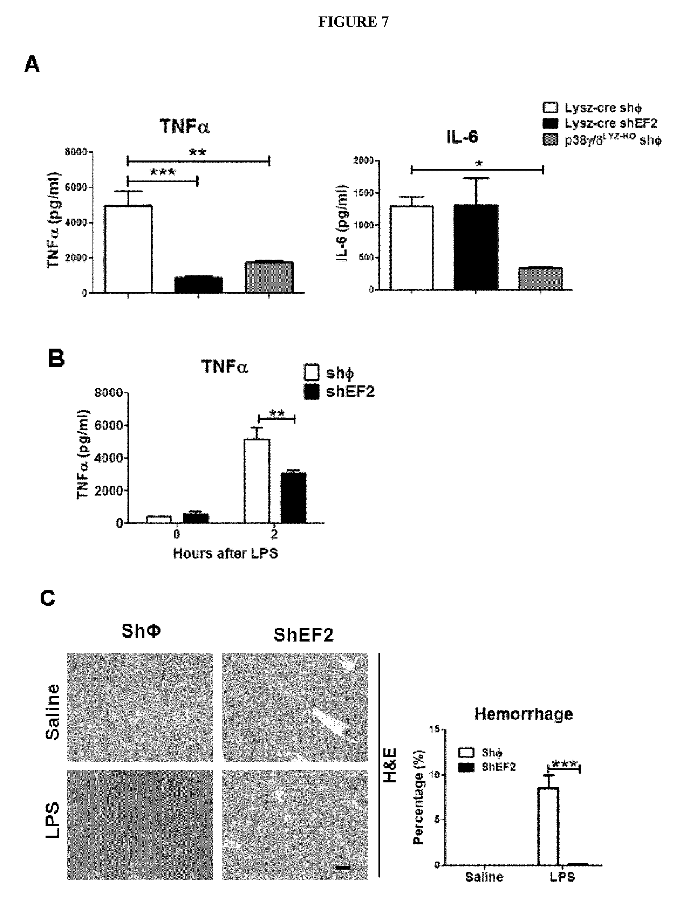

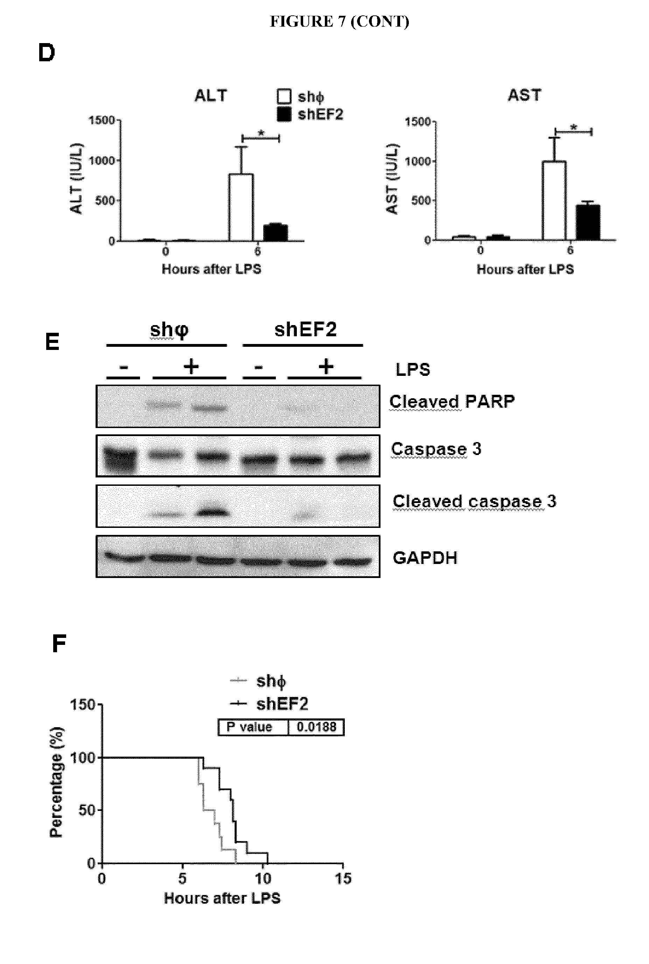

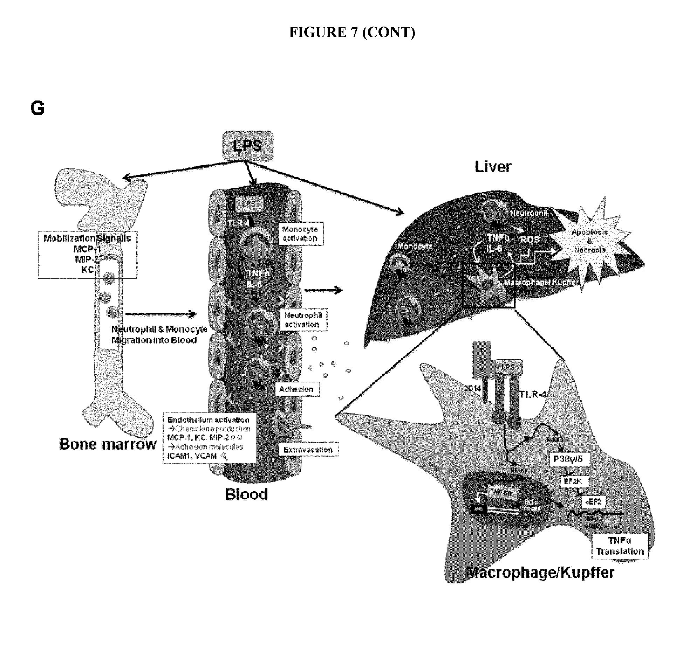

In summary, the present invention concludes that p38γ/δ in myeloid cells promote TNFα production by activating its translation without changes in mRNA levels. This is achieved by phosphorylation of eEF2K, releasing the inhibitory action of this kinase on eEF2. This post-transcriptional regulation is an important mechanism regulating cytokine secretion during the innate immune response and provides potential targets for the treatment of liver diseases, such as is demonstrated by the present invention (see **Example 10** and **Figure 7**), wherein it is shown that the inhibition of eEF2 /activation of eEF2k inhibits, at the same time, the expression/elongation of TNFα and, consequently, eEF2/eEF2k can be seen as strong therapeutic targets where drugs can be directed in order to treat TNFα-related-diseases.

### Brief description of the figures

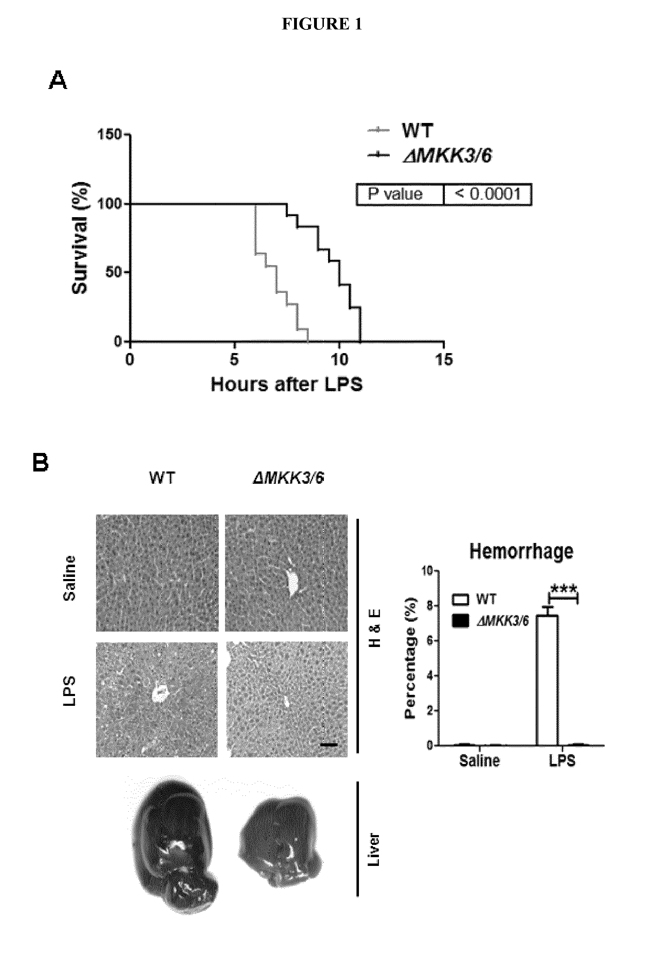

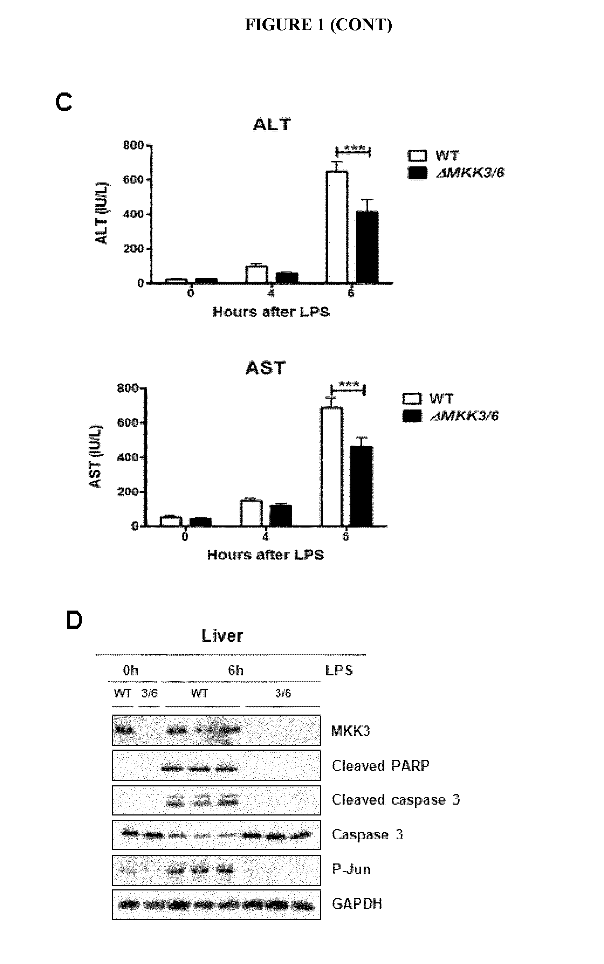

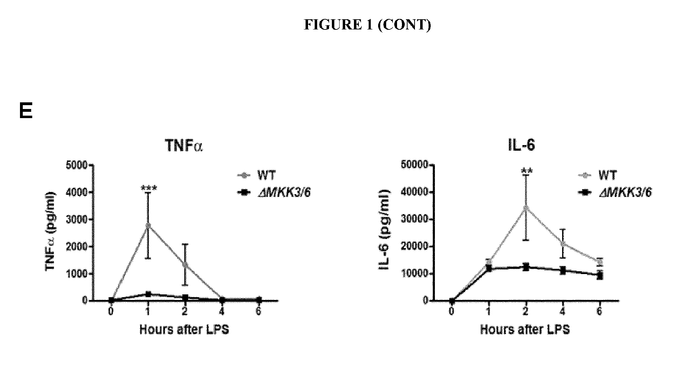

### Figure 1. ΔMKK3/6 mice are protected against LPS-induced liver damage.

Wild-type mice (WT) and *Mkk3*^{*-*/}*⁻ Mkk6*^{*-*/*+*} mice (Δ*MKK316*) were treated with D-Gal+LPS or saline.
(A) Survival curves after D-Gal+LPS injection (n=21). Survival curves were created with the Kaplan-Meier method and compared by Log-rank (Mantel-Cox) test.
(B) Livers were removed at 6 h post injection. Panels show representative H&E-stained liver sections (bar, 50µm) and livers. The chart presents hemorrhagic area as a percentage of the total area (n=6-8).
(C) Serum transaminase activity at 4 and 6 h post injection (n = 10).
(D) Liver extracts were examined by immunoblot with antibodies to cleaved PARP, cleaved caspase 3, caspase 3, phospho-Jun and GAPDH (n=6-8).
(E) ELISA analysis of serum TNFα and IL-6 at different times post injection (n=16). Data are means + SD. **P<0.01; ***P<0.001 (Two-way ANOVA coupled to Bonferroni post-tests).

**Example 2** gives a thoroughly explanation about the results illustrates in this **Figure 1****.**

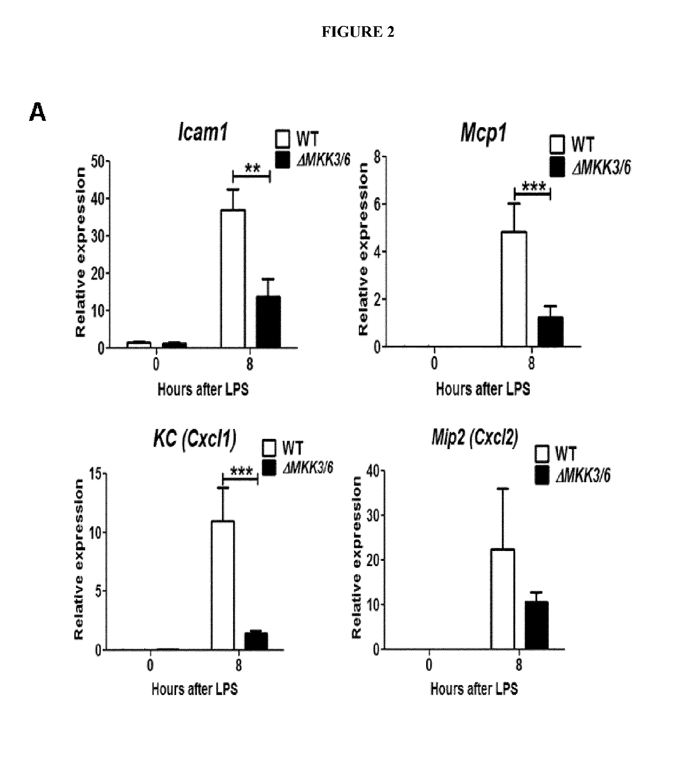

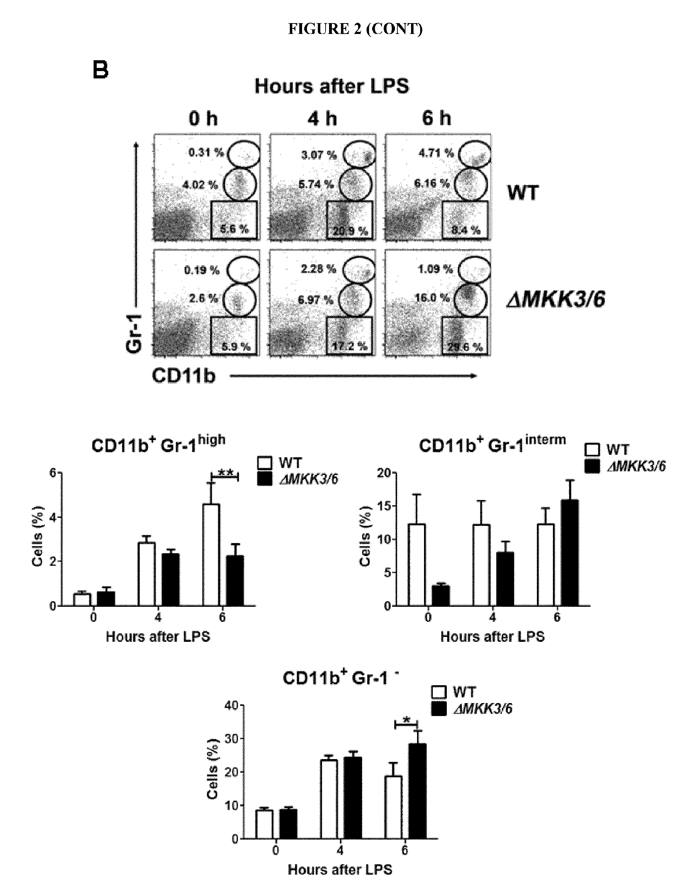

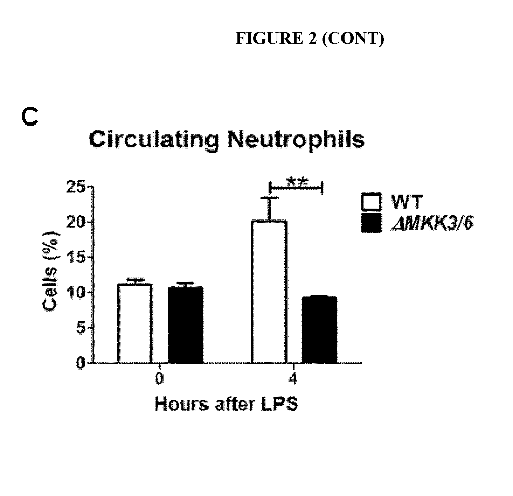

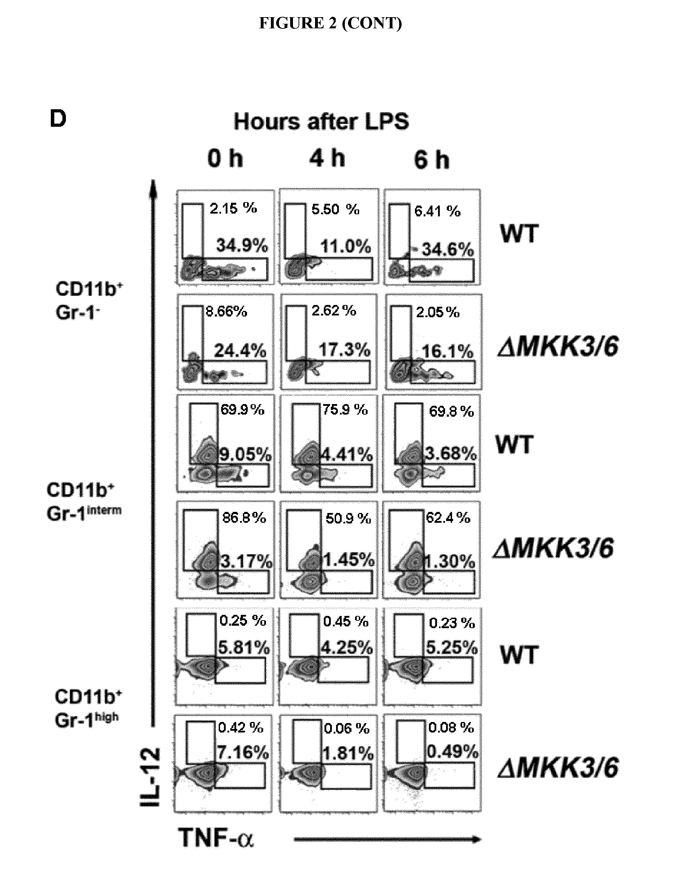

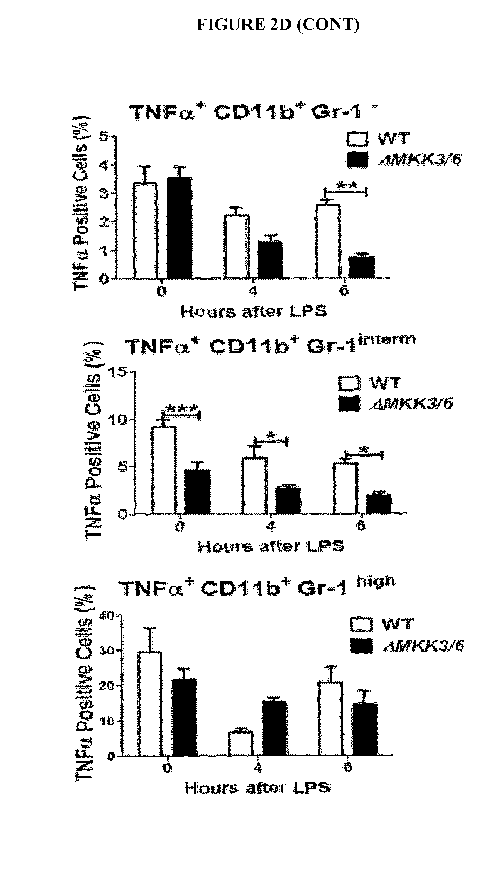

### Figure 2. ΔMKK3/6 mice have lower liver inflammation after LPS injection.

WT and Δ*MKK3*/*6* mice were treated with D-Gal+LPS or saline.
(A) Total RNA was extracted from livers 6 h after treatment, and chemokine mRNA levels determined by qRT-PCR. mRNA expression was normalized to *Gapdh* (n=5-8).
(B) Liver myeloid subsets (CD11b⁺ Gr-1^{high}, CD11b⁺ Gr-1^{intermediate}, CD11b⁺ Gr-1⁻) were assessed by flow cytometry of liver leukocytes isolated from WT and Δ*MKK3*/*6* mice 4 and 6 h after treatment. Representative dot plots are shown. Bar charts show each myeloid population as the percentage of total intra-hepatic leukocyte population (n=7).
(C) Neutrophils as a percentage of circulating leukocytes, measured in total blood 4 h post injection (n=5-8).
(D) TNFα and IL-12 production by liver myeloid subsets was analyzed by intracellular staining in neutrophils (CD11b⁺ Gr-1^{high}) monocytes (CD11b⁺ Gr-1^{intermediate}) and CD11b⁺ Gr-1⁻ myeloid cells isolated from WT and Δ*MKK3*/*6* mice 4 and 6 h post injection. Representative dot plots are shown for all treatment groups, and bar charts show TNFα-positive cells as the percentage of each myeloid population (n=7).

Data are means ± SD. **P<0.01; ***P<0.001 (two-way ANOVA coupled to Bonferroni post-tests).

**Examples 3** and **4** give a thoroughly explanation about the results illustrates in this **Figure 2****.**

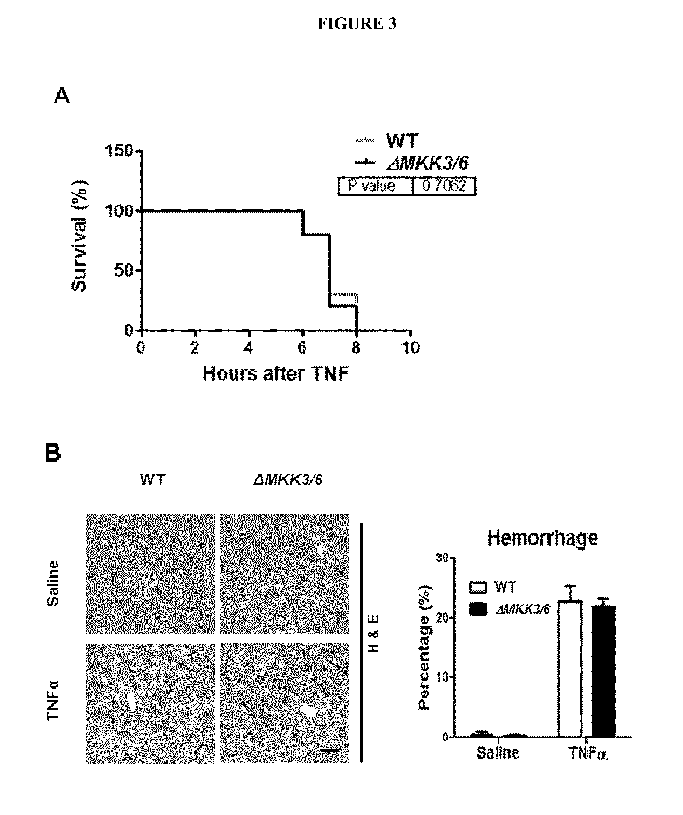

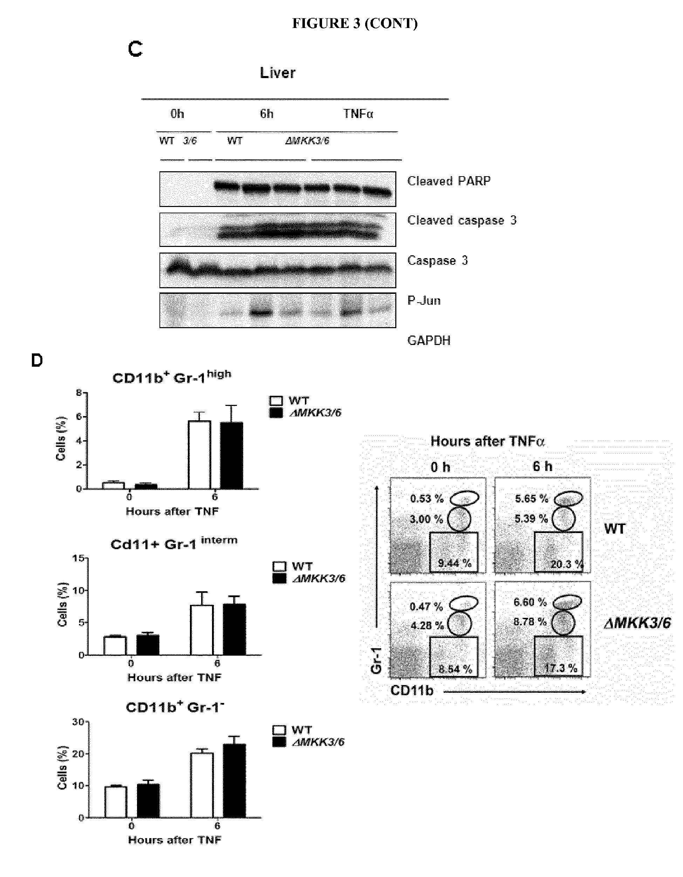

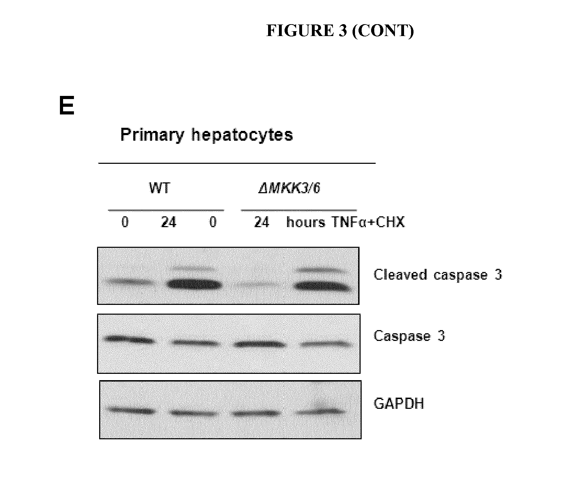

### Figure 3. ΔMKK3/6 mice are not protected against TNFα-induced liver damage.

WT and Δ*MKK3*/*6* mice were i.v. injected with 10 µg/kg TNFα plus 1g/kg D-Gal (TNFα/Gal) or with saline.
(A) Survival curves after TNFα/Gal injection (n = 13). Survival curves were created with the Kaplan-Meier method and compared by Log-rank (Mantel-Cox) test.
(B) Representative H&E-stained sections (scale bar 50µm) of livers extracted 6 h post injection. The chart presents hemorrhagic area as a percentage of the total area (n=8).
(C) Liver extracts were examined by immunoblot analysis for cleaved PARP, cleaved caspase 3, caspase 3, phospho-Jun and GAPDH (n=6-8).
(D) Liver myeloid subsets (CD11b⁺ Gr-1^{high}, CD11b⁺ Gr-1^{intermediate}, CD11b⁺ Gr-1⁻) were assessed by flow cytometry of liver leukocytes isolated from WT and *ΔMKK3*/*6* mice 4 and 6 h after treatment. Representative dot plots are shown. Bar charts show each myeloid subset as a percentage of the total intra-hepatic leukocyte population (n= 4-6).
(E) Immunoblot of cleaved caspase 3, caspase 3 and GAPDH in WT and Δ*MKK3*/*6* primary hepatocytes treated with TNF (20ng/ml) plus cycloheximide (100µg/ml).

Data are means ± SD. No significant differences were found (two-way ANOVA coupled to Bonferroni post-tests).

**Example 5** gives a thoroughly explanation about the results illustrates in this **Figure 3****.**

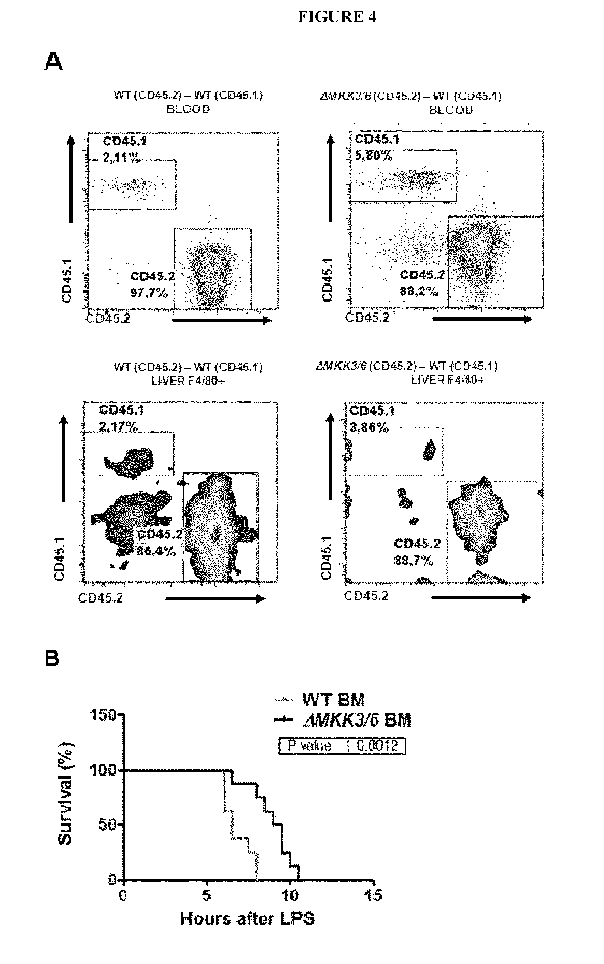

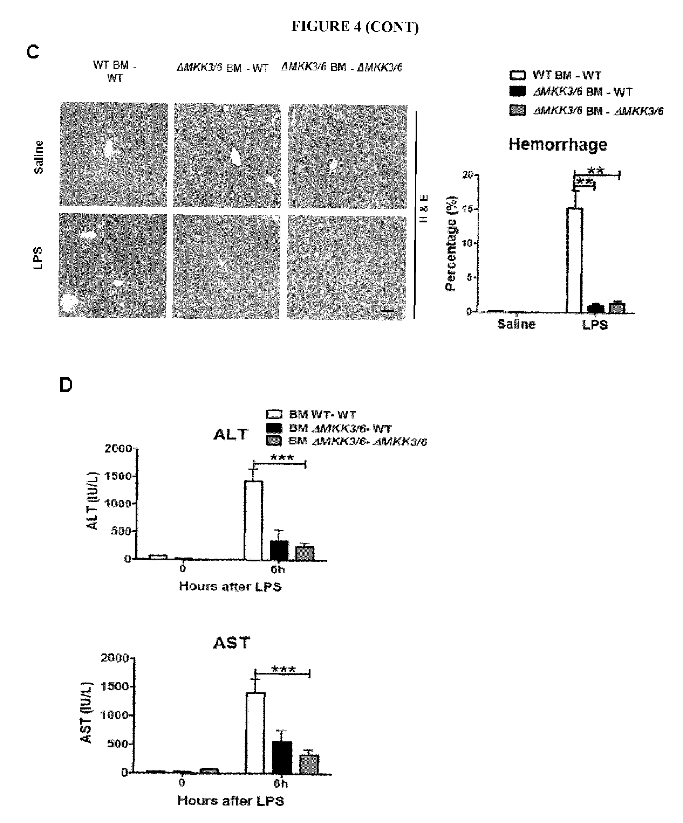

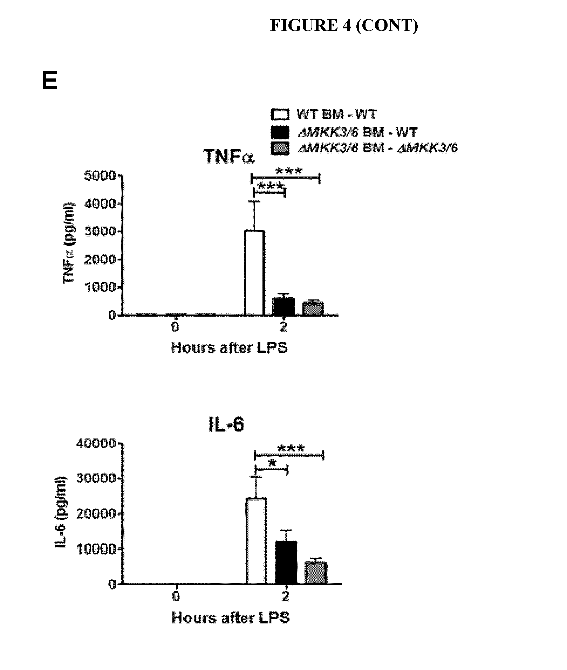

### Figure 4. ΔMKK3/6 hematopoietic cells protect mice against hepatitis.

Lethally-irradiated WT mice were reconstituted with bone marrow from WT or Δ*MKK3*/*6* mice. Mice were treated with D-Gal+LPS two months after transplantation.
(A) 2 x 10⁷ freshly-prepared CD45.2 whole bone marrow mononuclear cells were transplanted into lethally irradiated B6.SJL (CD45.1) mice, and engraftment by CD45.2 cells (%) was analyzed by antibody staining and FACS of peripheral blood after 4 months. Upper panel: representative FACS dot plot of CD45.1 and CD45.2 expression in cells isolated from the blood of transplanted mice. Lower panel: representative FACS dot plot of CD45.1 and CD45.2 expression in F4/80 positive cells isolated from the liver of transplanted mice (n=3).
(B) Survival curves after D-Gal+LPS injection (n = 10). Survival curves were created with the Kaplan-Meier method and compared by Log-rank (Mantel-Cox) test.
(C) Representative H&E-stained sections (scale bar 50µm) of livers extracted 6 h post injection. The chart presents hemorrhagic area as a percentage of the total area (n=5-8).
(D) Serum transaminase activity at 4 and 6 h post injection (n =5-8).
(E) ELISA of serum TNFα and IL-6 at different times post injection (n=5- 8).

Data are means ± SD. *P < 0.05; ***P<0.001 (two-way ANOVA coupled to Bonferroni post-tests).

**Example 6** gives a thoroughly explanation about the results illustrates in this **Figure 4****.**

### Figure 5. p38γ/δ^{Lyz-KO} mice are protected against LPS-induced liver damage.

p38γ^{Lyz-KO}, p38δ^{LVz-KO}, p38γ/δ^{Lyz-KO} and control Lyzs-cre transgenic mice were injected with D-Gal+LPS or saline.
(A) Mouse survival after D-Gal+LPS injection (n = 14). Survival curves were created with the Kaplan-Meier method and compared by Log-rank (Mantel-Cox) test.
(B) Livers were removed at 6 h post-injection. Panels show representative H&E-stained liver sections (bar, 50µm) and livers (n=5-8).
(C) Hemorrhagic area as a percentage of the total area on H&E-stained liver sections (n=5-8).
(D) Immunoblot analysis of liver extracts (n=5-8).
(E) Serum transaminase activity at 6 h post injection (n = 8-10).
(F) ELISA of serum TNFα and IL-6 at different times post injection (n = 10). #P< 0.01 for Lyzs-cre versus p38δ^{Lyz-KO} mice in TNFα; *P<0.001 for Lyzs-cre versus p38γ/δ^{Lyz-KO} mice for TNFα and for Lyzs-cre versus p38γ/δ^{Lyz-KO} or p38δ^{Lyz-KO} mice in IL-6 (n=6-8).
(G) Liver myeloid subsets (CD11b⁺ Gr-1^{high}, CD11b⁺ Gr-1^{intermediate}, CD11b⁺ Gr-1⁻) were assessed by flow cytometry of liver leukocytes isolated from from p38γ^{Lyz-KO}, p38δ^{LVz-KO}, p38γ/δ^{Lyz-KO} and control Lyzs-cre transgenic mice 6 h post injection. Representative dot plots are shown. Bar charts show each myeloid population as a percentage of the total intra-hepatic leukocyte population (mean ± SD; n=4-6). Circulating neutrophils in total blood were measured as a percentage of circulating leukocytes 4 h post injection (n=5-8).

Data are means ± SD. **P<0.01; ***P<0.001 (two-way ANOVA coupled to Bonferroni post-tests).

**Example 8** gives a thoroughly explanation about the results illustrates in this **Figure 5****.**

### Figure 6. p38γ/δ^{Lyz-KO} macrophages promote TNFα translation through phosphorylation of eEF2 kinase.

(A-D) BM-derived macrophages from p38γ/δ^{Lyz-KO} and Lyzs-cre mice were treated with LPS (10µg/ml) or TNFα (20ng/ml). Data were analyzed by two-way ANOVA coupled to Bonferroni post-tests.
(A) ELISA of TNFα and IL-6 in supernatants.
(B) qRT-PCR of *Tnf*α and *Il-6* mRNA normalized to *Gapdh,*
(C) Immunoblot of BM-derived macrophage (BMM) lysates.
(D) Flow cytometry of phospho-p38 in BM-derived macrophages stimulated with LPS and TNFα for 30 min. Intracellular phospho-p38 is depicted in blue; red corresponds to the isotype control.
(D-I) Cells were treated with LPS. *Tnf*α mRNA was detected by qRT-PCR in immunoprecipitates (1mg protein) obtained with anti-eEF2. mRNA amounts are expressed relative to the amount detected in control IgG immunoprecipitates. Data were analyzed by Student's t-test unless otherwise indicated.
(E) *Tnf*α mRNA in RAW 264.7 cell immunoprecipitates.
(F) *Tnf*α, *Hif1*α, and *Il-6* mRNA in RAW 264.7 cell immunoprecipitates. The presence of eEF2 was determined by immunoblot (upper panel) and quantified with ImageJ (one-way ANOVA coupled to Bonferroni post-tests).
(G) RAW 264.7 cells were pretreated with DMSO or BIRB796 (10µM) for 30 min before stimulation with LPS (60min). *Tnfα* mRNA was measured in immunoprecipitates.
(G-H) *Tnfα* mRNA in immunoprecipitates of BM-derived macrophages from Lyzs-cre and 38γ/δ^{Lyz-KO} mice (G) or WT or ΔMKK3/6 mice (H) after stimulation (60min). eEF2 in immunoprecipitates was detected by immunoblot (upper panel).

Data are means ± SD; (n = 4). *P < 0.05; **P<0.01; ***P<0.001.

**Examples 8 and 9** give a thoroughly explanation about the results illustrates in this **Figure 6****.**

### Figure 7. eEF2 controls TNFα elongation and its gene silencing protects against LPS-induced liver damage.

(A) BM-derived macrophages from Lyzs-cre and p38γ/δ^{Lyz-KO} mice were transduced with GFP⁺ lentivirus expressing eEf2 shRNA (shEF2) or with empty vector (shΦ). ELISA of TNFα and IL-6 in culture supernatants was performed 16 h after LPS (10µg/ml) stimulation. One-way ANOVA coupled to Bonferroni post-tests (n=6)
(B-F) WT mice were i.v. injected shEF2 or shΦ GFP⁺ lentiviral vectors. After seven days, mice were injected with D-Gal+LPS or saline. Data are means ± SD. *P < 0.05; **P<0.01; ***P<0.00 (two-way ANOVA coupled to Bonferroni post-tests).
(B) ELISA of TNFα and IL-6 in mouse serum 6h after treatment (n=16).
(C) Representative livers and H&E-stained sections (bar, 50µm) after removal at 6 h post-injection The chart presents hemorrhagic area as a percentage of the total area (n=6).
(D) Serum transaminase activity at 6 h post injection (n=6).
(E) Immunoblot analysis of liver extracts (n=6).
(F) Mouse survival after D-Gal+LPS injection (n = 10). Survival curves were created with the Kaplan-Meier method and compared by Log-rank (Mantel-Cox) test.
(G) The MKK3/6-p38γ/δ-eEF2K pathway is necessary for LPS-induced hepatitis. LPS induces leucocyte egress from bone marrow and cytokine/chemokine production in peripheral blood and by Kupffer cells. This leads to massive leucocyte infiltration in the liver and hepatocellular injury. TNFα plays a dominant role in LPS-induced liver injury. Activation of the MKK3/6-p38γ/δ-eEF2K pathway in macrophages/Kupffer cells controls TNFα production at the mRNA elongation level in acute inflammatory responses to LPS.

**Example 10** gives a thoroughly explanation about the results illustrates in this **Figure 7****.**

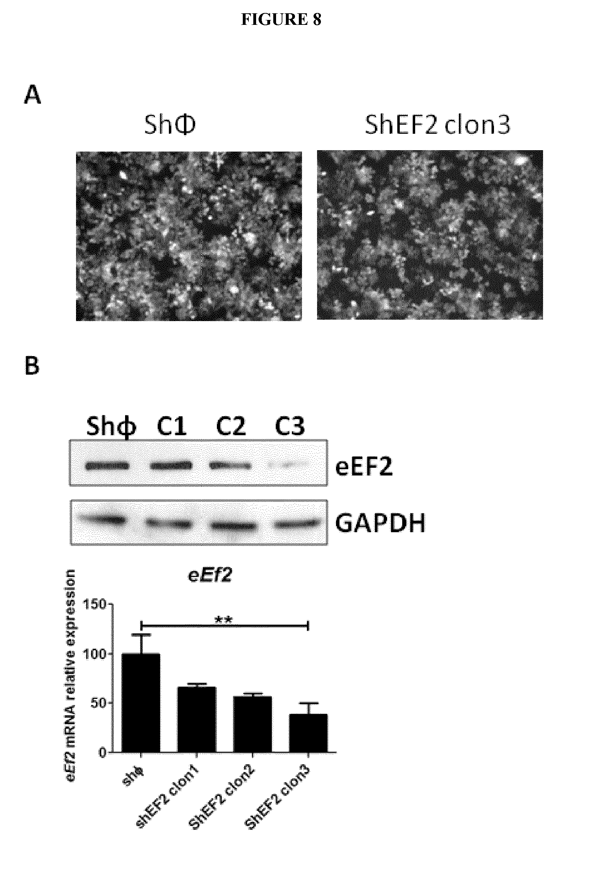

### Figure 8. This figure shows the effects of the three lentiviruses assayed in the present invention (C1 RMM4431-98752395, C2 RMM 4431-101293216 or C3 RMM 4431-101293680). All of them are able to reduce TNFα expression in a statistically significant way.

### EXAMPLES

### Results

### Example1. MKK3 and MKK6 collaborate in LPS induce hepatitis.

To study the role of p38MAPK signaling in hepatitis, the effect of D-Gal+LPS treatment on mice deficient for the upstream kinases MKK3 and MKK6 was examined. No differences in mortality where detected in *Mkk3*^{*-*/*-*} or *Mkk6*^{*-*/*-*} mice compared with wild type (WT) animals. However, *Mkk3*^{*-*/*-*} present less severe liver hemorrhage and *Mkk6*^{*-*/*-*} liver apoptosis was significantly reduced. Levels of alanine transaminase (ALT) and aspartate aminotrasferase (AST), two well established markers of hepatic necrosis, were not altered in *Mkk3*^{*-*/*-*} mice but were significantly reduced in *Mkk6*^{*-*/*-*} mice, thus showing an effect on liver necrosis.

The p38 MAPK pathway is a key regulator of the expression of inflammatory cytokines, including IL6 and TNFα, the main determinant of LPS-stimulated liver damage. Circulating levels of TNFα and IL6 after D-Gal+LPS injection did not differ between WT mice and the *Mkk3*^{*-*/*-*} or *Mkk6*^{*-*/*-*} mice. *In vitro* stimulation of isolated bone marrow (BM) macrophages from these mice with LPS also revealed no differences in proliferation rate or released TNFα and IL6 levels. Moreover, supernatants from WT, *Mkk3*^{*-*/*-*} and *Mkk6*^{*-*/*-*} macrophages induce apoptosis similarly in cultured hepatocytes, thus indicating that the partial protection against LPS-induced liver apoptosis was not due to circulating macrophages.

Neutrophil infiltration can cause mild to severe liver damage, and p38α was recently shown to control neutrophil cytokine production. It was examined liver myeloid leukocytes based on CD11b, Gr-1, F4/80 and Ly6C markers. Analysis of liver cell populations revealed that D-Gal+LPS injection increased liver infiltration by neutrophils (CD11b⁺ Gr-1^{high}) in WT mice, but this effect was impaired in *Mkk3*^{*-*/*-*} and *Mkk6*^{*-*/*-*} mice. To investigate the potential involvement of liver neutrophils and macrophages in the protection detected in *Mkk3*^{*-*/*-*} and *Mkk6*^{*-*/*-*} mice, leukocytes from the livers of WT, *Mkk3*^{*-*/*-*} and *Mkk6*^{*-*/*-*} mice were isolated and stimulated them with LPS. Whereas the percentage of neutrophils expressing TNFα was the same in all genotypes, the percentage of monocytes (CD11b⁺ Gr-1^{intermediate}) expressing TNFα was higher in WT mice than in *Mkk3*^{*-*/*-*} and *Mkk6*^{*-*/*-*} mice, indicating that the protection against LPS-induced liver apoptosis could be mediated by liver inflammatory monocytes.

### Example 2. Mkk3^{-/}⁻ Mkk6^{-/+} mice are protected against LPS-induced liver failure.

MKK3 and MKK6 show functional redundancy in the activation of p38 MAPK, and while single mutants are viable, doubly-deficient *Mkk3*^{*-*/}*⁻ Mkk6*^{*-*/*-*} and *Mkk3*^{*-*/}*⁺ Mkk6*^{*-*/*-*}mice die early in embryogenesis. To minimize interference from redundant actions *Mkk3*^{*-*/}*⁻ Mkk6*^{-/+} (ΔMKK3/6) mice were used. As expected, ΔMKK3/6 mice were protected against LPS-induced hepatotoxicity, showing milder hemorrhage and better survival than WT mice **(****Figure 1A** and **B).** Immunoblot analysis demonstrated that D-Gal+LPS injection activated caspase 3 and PARP cleavage in WT liver but not in ΔMKK3/6 livers (**Figure 1D****).** Moreover, circulating transaminases were reduced in ΔMKK3/6 mice, indicating a reduction in liver necrosis **(****Figure 1C****).** These data demonstrate that MKK3/6-deficiency severely limits LPS-stimulated hepatic apoptosis. Examination of serum TNFα after D-Gal+LPS injection revealed that the marked increase in TNFα concentration observed in WT and singly deficient mice was not detected in ΔMKK3/6 mice **(****Figure 1E****).** Serum IL6 was also lower in ΔMKK3/6 mice **(****Figure 1E****).** The loss of LPS-stimulated TNFα expression in ΔMKK3/6 mice might account for the finding that LPS-activation of JNK pathway in the livers of WT mice was not seen in ΔMKK3/6 mice **(****Figure 1D****).**

### Example 3. MKK3 and MKK6 are required for neutrophil migration after LPS injection.

The low TNFα production in ΔMKK3/6 mice could be a consequence of the reduced leukocyte infiltration in liver. Myeloid cell migration after D-Gal+LPS injection was analyzed. Quantification of liver chemokines showed that in WT mice LPS treatment increased mRNA expression of molecules important for migration of macrophages (MCP1 and ICAM) and neutrophils (KC, MIP2), whereas induction of these chemokines and adhesion molecules was decreased in the livers of ΔMKK3/6 mice (**Figure 2A****)**. Phenotypic characterization of liver-infiltrating leukocytes from control and D-Gal+LPS-treated livers revealed a significant increase in neutrophils in the inflammatory cell infiltrate of WT mice that was impaired in ΔMKK3/6 mice (**Figure 2B**), consistent with the reduced disease severity. However, the percentages of macrophages and monocytes did not differ between genotypes (**Figure 2B**). These findings correlated with lower RNA expression of neutrophil markers (*Elastase 2, Ly6G*) in the livers of LPS-injected ΔMKK3/6 mice.

The reduced liver neutrophil infiltration in ΔMKK3/6 mice could be due either to impaired neutrophil release from bone marrow or to a defect in cell migration. The levels of circulating neutrophils after LPS injection showed a sharp increase in WT mice that was absent in ΔMKK3/6 mice **(****Figure 2C****).** These data show that the reduced neutrophil infiltration in liver is in part caused by lowered neutrophil mobilization from the BM. To investigate whether the low circulating levels of TNFα in ΔMKK3/6 mice might be responsible for this alteration, it was injected TNFα + D-Gal into WT and ΔMKK3/6 mice, resulting in increased circulating neutrophil levels in both genotypes. These data indicate that the defect in migration is secondary to the low production of TNFα in ΔMKK3/6 mice.

### Example 4. MKK3 and MKK6 regulate TNFα production by macrophages.

To investigate the molecular mechanism underlying reduced circulating TNFα in ΔMKK3/6 mice, BM-derived macrophages from WT and ΔMKK3/6 mice were treated with LPS. Whereas treatment of WT macrophages with LPS activated JNK, ERK, and p38 MAPK, in ΔMKK3/6 macrophages the activation of p38 MAPK was impaired. The ΔMKK3/6 macrophages also secreted markedly reduced amounts of TNFα and IL6 compared with WT macrophages, confirming that MKK3/6-deficiency causes major defects in TNFα production. Further analysis showed that neutrophils and Kupffer cells, two other myeloid cell types implicated in liver damage, also secreted reduced amounts of TNFα and IL6. However, *in vivo* analysis indicated that although the percentage of liver neutrophils expressing TNFα after D-Gal+LPS injection was the same in both genotypes, the percentage of monocytes and macrophages expressing TNFα was higher in WT livers (Figure 2D).

To confirm that reduced cytokine production by liver macrophages is responsible for the protection against hepatocyte apoptosis in ΔMKK3/6 mice, primary hepatocytes were stimulated with conditioned medium from LPS-treated BM-derived macrophages. Conditioned medium from LPS-treated WT macrophages increased hepatocyte apoptosis, measured by cleavage of caspase 3 and PARP, whereas conditioned medium from LPS-treated ΔMKK3/6 macrophages had no effect.

### Example 5. MKK3 and MKK6 are not required for TNFα-induced liver damage.

This invention so far shows that MKK3/6-deficiency causes decreased TNFα production in a TNFα-dependent hepatitis model. This implies that ΔMKK3/6 mice are defective for TNFα secretion but not TNFα responsiveness. To test this, mice were treated with TNFα, finding that TNFα caused similar mortality and liver damage in WT and ΔMKK3/6 mice **(****Figure 3A and B****)**. Immunoblot analysis demonstrated that TNFα stimulated a similar degree of caspase 3 and PARP cleavage **(****Figure 3C****).** Moreover, TNFα induced the same levels of neutrophil liver infiltration in both genotypes **(****Figure 3D****)**. To confirm that the protection was due to defective cytokine production and not to a protective loss of responsiveness to TNFα, primary hepatocytes from WT or ΔMKK3/6 mice were treated with TNFα and the protein synthesis inhibitor cycloheximide (CHX). Both hepatocyte populations presented the same level of apoptosis **(****Figure 3E****).** These data demonstrate that ΔMKK3/6 mice do not exhibit resistance to TNFα-induced hepatitis and indicates that the primary protection against LPS-induced hepatitis in these mice is due to an inability to produce TNFα.

### Example 6. MKK3 and MKK6 are required in the hematopoietic compartment for LPS-induced liver damage.

The source of TNFα in mouse models of hepatitis is the hematopoietic compartment. Therefore, if defective TNFα expression accounts for the protection of ΔMKK3/6 mice against TNFα-dependent hepatitis, transplantation of the hematopoietic compartment of ΔMKK3/6 mice to immunodeficient donors should confer this protection. To test the role of hematopoietic cells, radiation chimeras were constructed by transplantating bone marrow from WT and ΔMKK3/6 donor mice into lethally irradiated congenic WT and ΔMKK3/6 recipients. Efficient reconstitution of B6.SJL (CD45.1) mice with bone marrow from WT C57BL/6J (CD45.2) or ΔMKK3/6 (CD45.2) mice was confirmed by flow cytometry analysis of peripheral blood leukocytes stained with antibodies to CD45.1/CD45.2. Moreover, analysis of Kupffer cells (F4/80⁺) in the liver of post-transplanted recipients showed that more than 90% of Kupffer cells originated from the donor mice **(****Figure 4A****).**

It was found that lethally-irradiated mice reconstituted with ΔMKK3/6 bone marrow were indeed protected against LPS-induced mortality and exhibited lower levels of LPS-stimulated serum TNFα and hepatic damage than mice reconstituted with WT bone marrow (**Figure 4B-E)**. These data confirm that MKK3 and MKK6 expressed in hematopoietic cells play a critical role in the expression of TNFα that leads to the development of hepatitis.

### Example 7. Inhibition of p38γ and δ protects against LPS induce hepatitis.

MKK3 and MKK6 are the main regulators of the α, β, γ and δ p38MAPK isoforms. To determine which isoforms mediate MKK3/MKK6-induced TNFα expression, it was analyzed the effect on D-Gal+LPS-induced liver damage of two inhibitors: SB203580, which inhibits p38α and p38β; and BIRB796, which inhibits all isoforms. Mice were treated with BIRB796, SB203580 or DMSO prior to LPS injection. The specific action of these inhibitors on MAPK pathway activity was firstly examined. Both compounds efficiently inhibited p38α/β, as shown by the loss of the phosphorylation of the known p38α substrate HSP27. In contrast, only BIRB796 was able to inhibit p38δ activation, as indicated by suppression of the phosphorylation of its substrate, eEF2K. No changes in ERK or JNK phosphorylation were observed. Injection of BIRB796 into WT mice protected against D-Gal+LPS-induced liver damage, whereas SB203580 exacerbated liver damage (hemorrhage, apoptosis and necrosis). Injection of BIRB796 also reduced serum levels of IL6 and TNFα in D-Gal+LPS-treated mice. These results suggest that LPS-induced liver injury might be mediated by p38γ and p38δ

### Example 8. Myeloid expression of p38γ and δ is required for LPS-induced hepatitis.

To investigate the role of p38γ and p38δ in LPS-induced hepatitis mice with myeloid-cell-specific ablation of these p38 isoforms were generated, separately and in combination. Mice carrying conditional loxP-flanked alleles for p38γ, p38δ, p38γ/δ (p38γ*^{fl}*, p38δ*^{fl}* and p38γ/δ*^{fl}* mice) were crossed with Lyzs-cre transgenic mice, yielding p38γ^{Lyz-KO}, p38δ ^{Lyz-KO} and p38γ/δ ^{yz-KO} mice. Immunoblot analysis confirmed efficient ablation of the appropriate kinases in macrophages, neutrophils and Kupffer cells.

Compared with control Lyzs-cre transgenic mice, p38δ ^{Lyz-KO} and p38γ/δ ^{Lyz-KO} mice showed a delay in mortality in response to injection of D-Gal+LPS (**Figure 5A****).** These mice were also protected against liver damage and apoptosis, showing milder liver hemorrhaging, reduced serum levels of ALT and AST, and reduced caspase 3 cleavage (**Figure 5B-E)**. Analysis of cytokines revealed significantly lower circulating TNFα and IL6 in LPS-injected p38δ^{Lyz-KO} and p38γ/δ^{Lyz-KO} mice than in similarly treated Lyzs-cre controls (**Figure 5F**). Other chemokines involved in leukocyte migration (MIP1α and MIP1β) were also reduced. In contrast, p38γ^{Lyz-KO} mice presented only a modest effect on circulating TNFα levels and no differences in the levels of other cytokines (**Figure 5F****).**

The phenotype found in p38γ/δ^{Lyz-KO} mice might reflect the function of these kinases in several myeloid cell populations implicated in D-Gal+LPS damage (such as neutrophils, Kupffer cells and macrophages). Analysis of these cell types in Lyzs-cre mice revealed expression of p38γ and δ in macrophages, predominant expression of p38δ in neutrophils, and exclusive expression of p38γ in Kupffer cells. Moreover, p38γ is strongly activated in Kupffer cells by LPS or TNFα treatment, as judged by the impaired p38 phosphorylation observed by FACS in Kupffer cells from p38γ/δ^{Lyz-KO}mice. Furthermore, analysis of phospho-p38 by western blot confirmed that p38γ was the isoform activated in these cells after LPS treatment. LPS and TNFα also activated p38γ and p38δ in macrophages (**Figure 6**), while in neutrophils p38α was the only isoform activated after either stimulus. Since neutrophils, Kupffer cells and macrophages express p38 γ, δ or both isoforms, any of these cell populations could have a role in the protection observed in p38γ/δ ^{Lyz-KO} mice. The different leukocyte populations that infiltrated the liver after D-Gal+LPS injection were analyzed. Analysis of liver-infiltrating leukocytes showed lower numbers of neutrophils in LPS-injected p38γ/δ^{Lyz-KO} mice than in similarly-treated Lyzs-cre mice. However, this reduction was not significant in LPS-injected p38δ^{Lyz-KO} mice (**Figure 5G**). None of the myeloid-specific p38-deficient mice showed alterations in the percentages of infiltrated macrophages or monocytes. Collectively, these data show that p38γ/δ^{Lyz-KO} mice are more strongly affected than either of the single conditional knockouts; however, p38δ ^{Lyz-KO} animals have a stronger phenotype than p38γ ^{Lyz-KO} mice, indicating partial redundancy between p38γ and p38δ, with a predominant role of p38δ. This overlapping action has been shown before, and might account for the limited protection observed in single conditional knockouts. p38γ/δ^{Lyz-KO} mice were used for further experiments. Further studies showed that the reduced neutrophil infiltration in liver is, in part, caused by lowered neutrophil mobilization from the BM (**Figure 5G**), probably due to the defective production of TNFα. Furthermore, measurement of TNFα-stimulated chemotaxis in a chamber assay revealed a slightly lower-than-control mobility in p38γ/δ^{Lyz-KO} neutrophils, and this effect was not increased by stimulation with a cytokine cocktail. Neutrophils produce several cytokines and this function can be controlled by p38α. Examination of cytokine production in the present invention showed that p38γ/δ^{Lyz-KO} neutrophils express slightly reduced levels of TNFα and IL6 after LPS treatment, but not sufficient to explain the phenotype observed.

To investigate whether the lower neutrophil infiltration in the livers of p38γ/δ^{Lyz-KO} mice could account for the protection observed, it was investigated whether neutrophil specific depletion in Lyzs-cre mice results in milder liver damage upon D-Gal+LPS treatment, similar to the phenotype of p38γ/δ^{Lyz-KO} mice. GR1 is highly expressed on neutrophils, and anti- GR1 antibody treatment has been shown to specifically deplete neutrophils (25). Treatment of mice with anti-GR1 one day before D-Gal+LPS injection completely depleted Grl+ neutrophils. Neutrophil depletion did not affect liver damage in Lyzs-cre mice, and neither did it abolish the protection observed in p38γ/δ^{Lyz-KO} as assessed by the levels of ALT and AST, cleaved caspase 3 and PARP, or liver damage observed in H&E-stained sections.

These data indicate that neutrophils do not play a dominant role in the protection against D-Gal+LPS-stimulated hepatic damage observed in p38γ/δ^{Lyz-KO} mice, and therefore indicate that a different myeloid cell type underlies this phenotype. Available evidence suggests an involvement of cytokine and chemokine production by activated Kupffer cells and infiltrated macrophages in the pathogenesis of liver damage (26). It was therefore examined cytokine production by macrophages and Kupffer cells upon LPS treatment. ELISA analysis demonstrated that LPS-induced production of TNFα and IL6 was lower in p38γ/δ^{Lyz-KO} macrophages and Kupffer cells than in Lyzs-cre control cells (**Figure 6A**). Moreover, only the supernatant from macrophages of p38γ/δ^{Lyz-KO} animals was unable to induce apoptosis in primary hepatocytes, and analysis of this supernatant indicated a defect in cytokine production (**Figure 6A**). Similarly, induction of apoptosis by supernatant from control Lyzs-cre macrophages was blocked by addition of anti-TNFα antibody, resulting in a protection similar to that obtained with p38γ/δ^{Lyz-KO} supernatant. This finding thus indicates that the low TNFα levels in p38γ/δ^{Lyz-KO} macrophages and Kupffer cells could account for the protection against D-Gal+LPS-induced liver damage.

### Example 9. p38γ and δ mediate TNFα translation in myeloid cells by phosphorylating eEF2K.

p38 MAPK can promote cytokine expression at the level of transcription, post-transcription (by promoting mRNA stability), and translation. *Il-6* mRNA expression was reduced in p38γ/δ^{Lyz-KO} macrophages, but levels of *Tnfα* mRNA were comparable to those in Lyzs-cre macrophages (**Figure 6B**). The stability of *Tnfα* mRNA also showed no difference between the two genotypes. The translational action of p38δ has been linked to eukaryotic elongation factor 2 (eEF2) kinase (the enzyme that inactivates eEF2). This kinase is inhibited by p38δ-mediated phosphorylation on Ser-359, and also by ribosomal protein S6 kinase-mediated phosphorylation on Ser-366. Since little is known about the regulation of eEF2K in macrophages, it was investigated the effect of p38γ/δ deletion on the phosphorylation of eEF2K and its downstream substrate eEF2. LPS stimulation of Lyzs-cre macrophages induced eEF2K phosphorylation on Ser359, but this was markedly impaired in p38γ/δ^{Lyz-KO} macrophages (**Figure 6C****)**. This observation was also corroborated in Kupffer cells. eEF2K phosphorylation in Lyzs-cre macrophages correlated with dephosphorylation of eEF2, implying activation of elongation. In contrast, p38γ/δ^{Lyz-KO} macrophages sustained high levels of eEF2 phosphorylation after the stimulus, suggesting that elongation is abolished.

To assess whether eEF2 controls TNFα elongation it was examined the binding of eEF2 to *Tnfα* mRNA in immunoprecipitates of mRNA-eEF2 complexes in RAW cells. RAW extracts were incubated with anti-eEF2 or an isotype control antibody, and the presence of *Tnfα* mRNA in the immunoprecipitates was examined by quantitative real-time PCR. High relative levels of TNFα mRNA were detected in the anti-eEF2 immunoprecipitate and this association was increased by LPS treatment (**Figure 6E and F)**. High mRNA levels for *Hif1*α*,* a known eEF2 target, were also detected in the anti-eEF2 immunoprecipitate (**Figure 6F**). In contrast, *Il-6* mRNA levels did not differ between the anti-eEF2 and isotype control immunoprecipitates (**Figure 6F****)**. Pretreatment of RAW cells with BIRB796 abolished the association of eEF2 with *Tnf*α mRNA (**Figure 6G****)**. Moreover, TNFα mRNA was detected in anti-eEF2 immunoprecipitates from LPS-treated Lyzs-cre or WT BM-derived macrophages but not in immunoprecipitates from similarly-treated p38γ/δ^{Lyz-KO} or ΔMKK3/6 macrophages (**Figure 6H-I).** Together, these data indicate that *Tnf*α mRNA is a selective target of eEF2, and that the MKK3/6-p38γ/δ pathway, by releasing eEF2K-mediated inhibition of eEF2 activity, promotes the translational elongation of TNFα in macrophages.

### Example 10. eEF2 controls TNFα translation elongation.

To confirm eEF2 control of TNFα translation elongation, a recombinant lentivirus expressing an eEF2 shRNA (shEF2) (C3 RMM 4431-101293680) was used which causes robust eEF2 knockdown in RAW cells. In macrophages, shEF2-mediated loss of eEF2 caused a statistically significant decrease in TNFα production as compared with TNFα production caused by loss of p38γ/δ (p<0.01 between both) (**Figure 7A****).** In contrast, p38γ/δ-dependent IL6 production was eEF2-independent (**Figure 7A****).** These data indicate that the expression of TNFα is controlled by eEF2, while the reduced IL6 production in p38γ/δ^{Lyz-KO} mice is caused by a different mechanism, most likely the loss of p38γ/δ-regulated *Il6* gene transcription.

To test the role of eEF2 in vivo, mice were injected with the shEF2 lentivirus or control shRNA lentivirus one week before exposure to DGal+LPS. Blood levels of TNFα were strongly reduced by the shEF2-mjected mice compared with controls (**Figure 7B****)**. Moreover, this reduction in TNFα was associated with protection against hepatic cell death, measured by cleavage of caspase 3 and PARP and protection against hepatic necrosis, measured by liver haemorrhage and blood levels of ALT and AST **(****Figure 7C-E).** Collectively, these data indicate that p38γ/δ MAPK proteins are required for the translation elongation of TNFα in macrophages mediated by phosphorylation of eEF2K and the activation of eEF2.

### Example 11. Screening assay. Method for identifying compounds able to inhibit eEF2 and/or activate eEF2 kinase.

This example is introduced to illustrate the screening assay carried out in the present invention, without disclosing all the compounds actually assayed (for practical reasons). Thus, the purpose of this example is showing how the shRNA used in the present invention to inhibit eEF2 and/or activate eEF2 kinase (see **Example 10)** was selected, giving clear basis to reproduce this assay with any other compound.

The screening assay is based on the *in vitro* differentiation of bone marrow cells from WT mice and mice lacking p38γ/δ into macrophages. WT bone marrow-derived macrophages (BMDM) were infected with lentiviruses expressing a control shRNA (Thermo Scientific pGIZP empty vector) (RHS4348) or lentiviruses expressing an shRNA to be assayed against *Eej2* (Thermo Scientific pGIZP shEF2) (RMM 4431-101293680). On the other hand, BMDM lacking p38γ/δ were infected with control lentiviruses (Thermo Scientific pGIZP empty). After transduction, cells are induced to produce TNFα through the stimulation with lipopolysacchride (LPS; CAS 93572-42-0).

Femurs and tibias were obtained from male mice between 8-12 weeks. Extracted bones were soaked in 70% EtOH for no more than 1 minute. Bone tips were cut off and bones flushed with DMEM media without serum, using a syringe (25G needle), to obtain the bone marrow. Then, cells were washed twice with DMEM (GIBCO) by 5 min centrifugation at 1700 rpm (810 x g) and resuspended by pipeting thoroughly in differentiation medium. Differentiation Medium: DMEM/20%FBS (GIBCO) with recombinant M-CSF (R&D; CAS 81627-83-0) added just prior to use (final concentration of 1.5 ng/ml). Bone marrow cells were counted and seeded at 1x10⁶ cell /ml in a special Petri dish for bacterias (Falcon 351005) in a final volume of 10ml of differentiation media. Plates were placed in an incubator at 37[deg.] C. in 5% CO2 (Day 0).

On day 3, 5 ml of fresh differentiation medium were added to each plate. At this moment, cells were infected with control lentiviruses (Thermo Scientific pGIZP empty genome) or with lentiviruses expressing a shRNA to be assayed against *Eef2* (Thermo Scientific pGIZP shEF2). The volume of virus (Vv) per plate was calculated for an MOI (multiplicity of infection) of 8. In this case, 8 x10⁷ transductional units (MOI x n° of cells seeded) were added to each plate.

On day 6, media from plates was removed and replaced with selection medium, (differentiation medium supplemented with 3 µg/ml puromycin). The selection medium was changed every two days.

On day 11, lentivirally-transfected BMDM were ready for the assay. Media was removed; cells were washed with PBS and PBS discarded. The plates were flushed thoroughly with a syringe filled with DMEM (GIBCO) without serum and a 25G needle attached.

Cells were counted and seeded at 0.5x10⁶ cell /ml in 48 well plates, using 0.5 ml/well of DMEM/20% FBS medium.

Cells were let sit for 12 hours in DMEM/20% FBS at 37[deg.] C. in 5% CO2. Media from wells was removed and replaced with stimulation medium (DMEM/5% FBS with 10µg/ml of LPS (SIGMA)) or control medium (DMEM/5% FBS without LPS). Supernatants were collected at 16 hours after stimulation and frozen until measurement (-80°C).

TNFα levels in the supernatants were determined by Luminex assay (Biorad). For that aim, the standard curve provided with the *Biorad* kit was used. TNFα levels in the supernatants from non-stimulated cells (0µg/ml LPS) were lower than the kit's detection limit. However, WT BMDM infected with control lentiviruses secreted high levels of TNFα after LPS stimulation (Average 5000 pg/ml. Min 4000 y max 5800). The levels of TNFα in the supernatants from WT BMDM infected with lentiviruses expressing a shRNA assayed against *Eef2* were decrease up to 5 times (Average 900 pg/ml min 750 max 1200) compared with the levels from the WT cells infected with control lentiviruses. TNFα levels in the supernatants from BMDM lacking p38γ/δ were reduced two times compared with the levels from the WT cells infected with control lentiviruses (Average 1750 pg/ml min 1700 max 1800).

**In vivo assay:** Male mice *C57BL6J* 8 weeks of age were tail vein injected with 200µl of lentiviral particles (pGIZP empty (RHS4348) or pGIZP.shEF2 (RMM 4431-101293680) lentivirus from Thermo Scientific) resuspended in PBS (8 x10⁸ transductional units). Seven days after infection, mice were I.P injected with 50µg/kg *E. coli* 0111:B lipopolysacchride (LPS) (Sigma) plus 1g/kg N-acetyl-galactosamine (D-Gal; CAS Num. 1811-31-0) (Sigma). After two hours, blood (100ml) was collected from the check in EDTA tripotassium salt tubes (Microvette ®). Plasma was extracted by centrifugation and frozen at -80C until its use. The amount of TNFα was determinate by a Luminex assay (Biorad) using the standard curve provided with the kit. Mice infected with pGIZP empty lentivirus have (5300 pg/ml) while mice infected with pGIZP. shEF2 virus have (Average 3000 pg/ml).

### Methods

### Example 12. Mice.

*Mkk3*^{*-*/*-*} (B6.129-*Map2k3^{tm1Flv}*), *Mkk6^{-l-}* (B6.129-*Map2k6^{tm1Flv}*) were as previously described. p38γ (B6.129-*Mapk12^{tm1}*) and p38δ (B6.129-*Mapk13^{tm1}*) mice were crossed with B6.129P2-*Lyz2tm1(cre)Ifo*/J and back-crossed for ten generations to the C57BL/6J background (The Jackson Labs) and genotyped by PCR analysis of genomic DNA. Radiation chimeras were generated by exposing recipient mice to two doses of ionizing radiation (525 Gy) and reconstituting them with 2x10⁷ donor bone marrow cells by injection into the tail vein. Hepatitis was induced by i.p. injection with 50µg/kg *E. coli* 0111:B lipopolysacchride (LPS) (Sigma) plus 1g/kg N-acetyl-galactosamine (D-Gal) (Sigma) or by i.v. injection with 10µg/kg TNFα (R&D Systems) plus 1g/kg GalN. When required, inhibitors were administered by i.p. injection at 15mg/kg as previously reported (27, 28). For in vivo neutrophil depletion, 300 µg/mouse of anti-Gr1 or the same volume of saline were i.v. injected 24 h before challenge with LPS. Specificity of the depletion of cell populations was determined by flow cytometry of blood samples collected during the experiment.

### Example 13. Lentivirus vector production and infection of mice.

Lentiviruses were produced as described (29). Transient calcium phosphate cotransfection of HEK-293 cells was done with the pGIZP empty or pGIZP.shEF2 vector from Thermo scientific together with pΔ8.9 and pVSV-G. The supernatants containing the LV particles was collected 48 and 72 hours after removal of the calcium phosphate precipitate, and were centrifuged at 700g, 4°C for 10 min and concentrated (165x) by ultracentrifugation for 2 hours at 121 986g at 4°C (Ultraclear Tubes, SW28 rotor and Optima L-100 XP Ultracentrifuge; Beckman). Viruses were collected by adding cold sterile PBS and were titrated by quantitative PCR (qPCR).

Mice were injected in the tail vein with 200µl lentiviral particles suspended in PBS. Seven days after infection, mice were injected with LPS+D-Gal.

### Example 14. Cell culture.

Primary BM-derived macrophages were prepared and cultured as described previously. To isolate primary Kupffer cells from mouse livers we performed collagenase perfusion and differential centrifugation using Percoll (Pharmacia, Uppsala, Sweden). Bone marrow neutrophils were purified by positive selection using biotinylated GR-1 (BD Pharmingen) and MACS streptavidin microbeads (Miltenyi Biotec). Neutrophil purity was ≥95% as assessed by flow cytometry. Cytokines in the culture medium were measured by multiplexed ELISA using a Luminex 200 analyzer (Millipore) and a mouse cytokine kit (Millipore). For primary hepatocyte isolation, liver parenchymal cells were prepared from 8-to-12-week-old anesthetized mice by *in situ* collagenase perfusion of the liver. Primary monolayer cultures were established by plating 1.5 x 10⁵ viable cells/cm² in 6-well collagen-IV-coated plates. After 16 h, primary hepatocytes were stimulated for 24 h with TNFα (20ng/ml) plus cycloheximide (100µg/ml) or with 500µl of macrophage-conditioned medium for 12h. Neutrophil migration assays were performed using BD Falcon FluoroBlok 96 multiwell insert systems. Neutrophils were stained for 1h with 1.5µM calcein AM. Then, 1 x 10⁵ cells were plated per well in the upper chamber in 50 µl of culture medium without FBS. Lower chambers contained 200µl of culture medium alone or supplemented with with the chemoatractant (TNFα 20ng/ml or 1/10 cytokine cocktail). Fluorescence emission was measured at different time points with a fluorescent plate reader with bottom-reading capability (Thermo Scientific Fluoroskan Ascent).

### Example 15. Serum analysis.

Serum activities of alanine transaminase (ALT) and aspartate aminotrasferase (AST) were measured using the ALT and AST Reagent Kit (Biosystems Reagents) with a Benchmark Plus Microplate Spectrophotometer. Serum concentrations of cytokines were measured by multiplexed ELISA with a Luminex 200 analyzer (Millipore).

### Example 16. Biochemical analysis.

Tissue extracts were prepared using Triton lysis buffer (20 mM Tris [pH 7.4], 1% Triton X-100, 10% glycerol, 137mM NaCl, 2mM EDTA, 25mM β-glycerophosphate, 1 mM sodium orthovanadate, 1mM phenylmethylsulfonyl fluoride, and 10µg/mL aprotinin and leupeptin). Extracts (30µg protein) and immunoprecipitates (prepared from 2 mg protein) were examined by protein immunoblot analysis with antibodies to ERK1/2, phospho-ERK1/2, phospho-MKK3/6, p38α MAPK, phospho-p38, phospho-hsp27, phospho-JNK1/2, JNK1/2, caspase 3, cleaved caspase 3, cleaved PARP and phospho-eEF2 kinase (Ser366), all from Cell Signaling and MKK3 (Pharmingen), MKK6 (Stressgen), and GAPDH and eEF2 (Santa Cruz Biotechnology). The anti-p38γ and p38δ antibodies were raised against the peptides which correspond to the C termini of the proteins.

### Example 17. Immunohistochemistry.

Livers were fixed in 4% paraformaldehyde, processed, and embedded in paraffin. Sections (5 µm) were stained with hematoxylin and eosin (H&E).

### Example 18. Isolation of liver-infiltrating mononuclear leukocytes

Mouse livers were collected and a single-cell suspension was obtained and passed through a 70-µm strainer. Leukocytes were collected from the interphase of centrifuged Ficoll gradients. Cells were counted with a CASY Cell Counter. Cells (10⁶) cells were labeled by surface staining (PE-conjugated anti-CD11b and PerCP-conjugated anti-Gr-1, from Invitrogen), and another 10⁶ cells were stimulated in vitro with LPS (10µg/ml) plus brefeldin A which inhibits protein transport from ER to Golgi, leading to the accumulation of protein inside the ER (BD GolgiPlug™) (1:1000) for 2 h. The LPS-stimulated cells were then labeled by surface staining (PE-conjugated anti-CD11b and PerCP-conjugated anti-Gr-1, from Invitrogen), fixed, permeabilized, and stained for intracellular TNFα (APC-conjugated anti-TNFα from BD Biosciences) and IL-12 (PE-conjugated anti-IL-12 from BD Biosciences). Flow cytometry was performed with a FACScan cytofluorometer (FACS Canto BD) and data were examined using FlowJo software.

### Example 19. Phospho-p38 FACS analysis.

Primary myeloid cells were isolated (neutrophils, bone marrow macrophages and Kupffer cells) and stimulated for 30 minutes with LPS (10µg/ml). Cells were fixed overnight in 70% methanol at -20°C and stained with anti-phospho-p38 antibody (Cell Signaling) or control rabbit IgG followed by FITC-anti-rabbit IgG. Signal was detected by flow cytometry.

### Example 20. RNA-immunoprecipitation assay.

RAW 264.7 cells (Mouse leukaemic monocyte macrophage cell line) were stimulated with LPS (10µg/ml) and RNA-immunoprecipitation assays (RIPs) were performed using antibody against eEF2 (Santa Cruz Biotechnology). The efficiency of immunoprecipitation was checked by immunoblot analysis. RNA was isolated from the input lysate, isotype control immunoprecipitate, and the anti-eEF2 immunoprecipitate. After reverse transcription, the levels of TNFα mRNA were determined by qRT-PCR. Relative mRNA levels were calculated by subtracting background from the level of the target mRNA and then using the standard Δ-Δ C_{T} formula.

### Example 21. qRT-PCR.

The expression of mRNA was examined by quantitative RT-PCR using a 7900 Fast Real Time thermocycler and FAST SYBR GREEN assays (Applied Biosystems). Relative mRNA expression was normalized to *Gapdh* mRNA measured in each sample. *Hif-1*α, *Gr-1, KC* (*Cxcl1*), *Mip-2* (*Cxcl2*), *Mcp-1* and *Icam-1* were amplified using the primers.

Differences between groups were examined for statistical significance using Student's t-test or analysis of variance (ANOVA) coupled to the Bonferroni post-test. Kaplan-Meier analysis of survival was performed using the log-rank test. Animal studies were approved by the local ethics committee and by the Institutional Animal Care and Use Committee (IACUC) of the University of Massachusetts Medical School. All animal procedures conformed to EU Directive 86/609/EEC and Recommendation 2007/526/EC regarding the protection of animals used for experimental and other scientific purposes, enacted under Spanish law 1201/2005.

### BIBLIOGRAPHY

1. Kim, C., Sano, Y., Todorova, K., Carlson, B.A., Arpa, L., Celada, A., Lawrence, T., Otsu, K., Brissette, J.L., Arthur, J.S., et al. 2008. The kinase p38 alpha serves cell type-specific inflammatory functions in skin injury and coordinates pro- and anti-inflammatory gene expression. Nat Immunol 9:1019-1027.
2. Wu, Z., Han, M., Chen, T., Yan, W., and Ning, Q. 2010. Acute liver failure: mechanisms of immune-mediated liver injury. Liver Int 30:782-794.
3. Antoniades, C.G., Berry, P.A., Wendon, J.A., and Vergani, D. 2008. The importance of immune dysfunction in determining outcome in acute liver failure. J Hepatol 49:845-861.
4. Popa, C., Netea, M.G., van Riel, P.L., van der Meer, J.W., and Stalenhoef, A.F. 2007. The role of TNF-alpha in chronic inflammatory conditions, intermediary metabolism, and cardiovascular risk. JLipid Res 48:751-762.
5. Wajant, H., Pfizenmaier, K., and Scheurich, P. 2003. Tumor necrosis factor signaling. Cell Death Differ 10:45-65.
6. Bradley, J.R. 2008. TNF-mediated inflammatory disease. J Pathol 214:149-160.
7. Schwabe, R.F., and Brenner, D.A. 2006. Mechanisms of Liver Injury. I. TNF-alpha-induced liver injury: role of IKK, JNK, and ROS pathways. Am J Physiol Gastrointest Liver Physiol 290:G583-589.
8. Pfeffer, K.D., Huecksteadt, T.P., and Hoidal, J.R. 1993. Expression and regulation of tumor necrosis factor in macrophages from cystic fibrosis patients. Am J Respir Cell Mol Biol 9:511-519.
9. Pasparakis, M., Alexopoulou, L., Douni, E., and Kollias, G. 1996. Tumour necrosis factors in immune regulation: everything that's interesting is...new! Cytokine Growth Factor Rev 7:223-229.
10. Nebreda, A.R., and Porras, A. 2000. p38 MAP kinases: beyond the stress response. Trends Biochem Sci 25:257-260.
11. Schieven, G.L. 2005. The biology of p38 kinase: a central role in inflammation. Curr Top Med Chem 5:921-928.
12. Cuenda, A., and Rousseau, S. 2007. p38 MAP-kinases pathway regulation, function and role in human diseases. Biochim Biophys Acta 1773:1358-1375.
13. Kyriakis, J.M., and Avruch, J. 2001. Mammalian mitogen-activated protein kinase signal transduction pathways activated by stress and inflammation. Physiol Rev 81:807-869.
14. Brancho, D., Tanaka, N., Jaeschke, A., Ventura, J.J., Kelkar, N., Tanaka, Y., Kyuuma, M., Takeshita, T., Flavell, R.A., and Davis, R.J. 2003. Mechanism of p38 MAP kinase activation in vivo. Genes Dev 17:1969-1978.
15. Remy, G., Risco, A.M., Inesta-Vaquera, F.A., Gonzalez-Teran, B., Sabio, G., Davis, R.J., and Cuenda, A. 2010. Differential activation of p38MAPK isoforms by MKK6 and MKK3. Cell Signal 22:660-667.
16. Heinrichsdorff, J., Luedde, T., Perdiguero, E., Nebreda, A.R., and Pasparakis, M. 2008. p38 alpha MAPK inhibits JNK activation and collaborates with IkappaB kinase 2 to prevent endotoxin-induced liver failure. EMBO Rep 9:1048-1054.
17. Hui, L., Bakiri, L., Mairhorfer, A., Schweifer, N., Haslinger, C., Kenner, L., Komnenovic, V., Scheuch, H., Beug, H., and Wagner, E.F. 2007. p38alpha suppresses normal and cancer cell proliferation by antagonizing the JNK-c-Jun pathway. Nat Genet 39:741-749.
18. Beardmore, V.A., Hinton, H.J., Eftychi, C., Apostolaki, M., Armaka, M., Darragh, J., McIlrath, J., Carr, J.M., Armit, L.J., Clacher, C., et al. 2005. Generation and characterization of p38beta (maps11) gene-targeted mice. Mol Cell Biol 25:10454-10464.
19. Knebel, A., Morrice, N., and Cohen, P. 2001. A novel method to identify protein kinase substrates: eEF2 kinase is phosphorylated and inhibited by SAPK4/p38delta. EMBO J 20:4360-4369.
20. Jung, G.A., Shin, B.S., Jang, Y.S., Sohn, J.B., Woo, S.R., Kim, J.E., Choi, G., Lee, K.M., Min, B.H., Lee, K.H., et al. 2011. Methylation of eukaryotic elongation factor 2 induced by basic fibroblast growth factor via mitogen-activated protein kinase. Exp Mol Med 43:550-560.
21. Browne, G.J., and Proud, C.G. 2002. Regulation of peptide-chain elongation in mammalian cells. Eur J Biochem 269:5360-5368.
22. Wang, L., Wang, X., and Proud, C.G. 2000. Activation of mRNA translation in rat cardiac myocytes by insulin involves multiple rapamycin-sensitive steps. Am J Physiol Heart Circ Physiol 278:H1056-1068.
23. Knebel, A., Haydon, C.E., Morrice, N., and Cohen, P. 2002. Stress-induced regulation of eukaryotic elongation factor 2 kinase by SB 203580-sensitive and -insensitive pathways. Biochem J 367:525-532.
24. Schreiber, S., Feagan, B., D'Haens, G., Colombel, J.F., Geboes, K., Yurcov, M., Isakov, V., Golovenko, O., Bernstein, C.N., Ludwig, D., et al. 2006. Oral p38 mitogen-activated protein kinase inhibition with BIRB 796 for active Crohn's disease: a randomized, double-blind, placebo-controlled trial. Clin Gastroenterol Hepatol 4:325-334.
25. Jonsson, F., Mancardi, D.A., Kita, Y., Karasuyama, H., Iannascoli, B., Van Rooijen, N., Shimizu, T., Daeron, M., and Bruhns, P. 2011. Mouse and human neutrophils induce anaphylaxis. J Clin Invest 121:1484-1496.
26. Kolios, G., Valatas, V., and Kouroumalis, E. 2006. Role of Kupffer cells in the pathogenesis of liver disease. World J Gastroenterol 12:7413-7420.
27. Chopra, P., Kulkarni, O., Gupta, S., Bajpai, M., Kanoje, V., Banerjee, M., Bansal, V., Visaga, S., Chatterjee, M., Chaira, T., et al. 2010. Pharmacological profile of AW-814141, a novel, potent, selective and orally active inhibitor of p38 MAP kinase. Int Immunopharmacol 10:467-473.
28. Sukhtankar, D., Okun, A., Chandramouli, A., Nelson, M.A., Vanderah, T.W., Cress, A.E., Porreca, F., and King, T. 2011. Inhibition of p38-MAPK signaling pathway attenuates breast cancer induced bone pain and disease progression in a murine model of cancer-induced bone pain. Mol Pain 7:81.
29. Urso, K., Alfranca, A., Martinez-Martinez, S., Escolano, A., Ortega, I., Rodriguez, A., and Redondo, J.M. 2011. NFATc3 regulates the transcription of genes involved in T-cell activation and angiogenesis. Blood 118:795-803.

## Claims

1. *In vitro* method for identifying compounds able to inhibit eEF2 and/or activate eEF2 kinase that comprises contacting said compounds with cells expressing eEF2, and determining whether TNF-alpha is infra-expressed as compared with the control.

2. Inhibitor of eEF2 and/or activator of eEF2 kinase for use in the treatment of TNF-alpha-related diseases.

3. Inhibitor of eEF2 and/or activator of eEF2 kinase, according to claim 2, for use in the treatment of TNF-alpha-related diseases selected from: liver diseases, rheumatoid arthritis, ankylosing spondylitis, psoriasis, ischaemic brain injury, traumatic injury, cardiovascular diseases, respiratory diseases, renal diseases or diabetes.

4. Inhibitor of eEF2 and/or activator of eEF2 kinase, according to claim 3, for use in the treatment of LPS-induced liver diseases, cardiovascular diseases selected from: atherosclerosis, myocardial infarction, heart failure, myocarditis and cardiac allograft rejection; respiratory diseases selected from: chronic bronchitis, obstructive pulmonary disease, acute respiratory distress syndrome and asthma; or renal diseases selected from: ischaemic renal injury, renal transplant rejection and glomerulonephritis.

5. Inhibitor of eEF2 for use in the treatment of TNF-alpha-related diseases, according to the claim 2, selected from: shEF2, sordarin or ciclohexamide.

6. Activator of eEF2 kinase for use in the treatment of TNF-alpha-related diseases, according to the claim 2, which is BIRB796.

7. Pharmaceutical composition comprising an inhibitor of eEF2 and/or activator of eEF2 kinase and any pharmaceutically acceptable carrier, for use in the treatment of TNF-alpha-related diseases.
